# EUROPEAN PATENT SPECIFICATION

(11) **EP 1 618 193 B1**
(45) Date of publication and mention of the grant of the patent: **07.05.2008**
(21) Application number: 04726971.7
(22) Date of filing: 13.04.2004
(51) Int. Cl.: C12N 15/09, C12P 7/64

(54) **USE OF GENES FOR INCREASING THE OIL CONTENT IN PLANTS**
VERWENDUNG VON GENEN ZUR ERHÖHUNG DES ÖLGEHALTES IN PFLANZEN
UTILISATION DE GENES POUR AUGMENTER LA TENEUR EN HUILE DANS LES PLANTES

(30) Priority: 16.04.2003 EP 03008909
(43) Date of publication of application: 25.01.2006
(73) Proprietor: BASF Plant Science GmbH, 67056 Ludwigshafen (DE)
(72) Inventor: CIRPUS, Petra, 68163 Mannheim (DE); OSWALD, Oliver, 67063 Ludwigshafen (DE); RONNE, Hans, S-75654 Uppsala (SE); DAHLQVIST, Anders, S-24466 Furulund (SE); LENMAN, Marit, S-22359 Lund (SE); NEAL, Andrea, Chapel Hill, NC 27514 (US); STAHL, Ulf, 75324 Uppsala (SE); LIU, Tao, S-22241 Lund (SE); BANAS, Antoni, PL-08-110 Siedlce (PL); WIBERG, Eva, S-75652 Uppsala (SE)
(74) Representative: Pressler, Uwe
(86) International application number: PCT/EP2004/003845
(87) International publication number: WO 2004/092367

(56) References cited:
- WO-A-00/18889
- JAIN R K ET AL: "ENHANCEMENT OF SEED SOIL CONTENT BY EXPRESSION OF GLYCEROL-3-PHOSPHATE ACYLTRANSFERASE GENES" BIOCHEMICAL SOCIETY TRANSACTIONS, COLCHESTER, ESSEX, GB, vol. 28, no. PART 6, December 2000 (2000-12), pages 958-961, XP009018636 ISSN: 0300-5127
- JAKO C ET AL: "Seed-specific over-expression of an Arabidopsis cDNA encoding a diacylglycerol acyltransferase enhances seed oil content and seed weight" PLANT PHYSIOLOGY, AMERICAN SOCIETY OF PLANT PHYSIOLOGISTS, ROCKVILLE, MD, US, vol. 126, no. 2, June 2001 (2001-06), pages 861-874, XP002242963 ISSN: 0032-0889
- TAYLOR D C ET AL: "FIELD TESTING OF TRANSGENIC RAPESEED CV. HERO TRANSFORMED WITH A YEAST SN-2 ACYLTRANSFERASE RESULTS IN INCREASED OIL CONTENT, ERUCIC ACID CONTENT AND SEED YIELD" MOLECULAR BREEDING, KLUWER ACADEMIC PUBLISHERS, DORDRECHT, NL, vol. 8, no. 4, 2001, pages 317-322, XP009009613 ISSN: 1380-3743
- DATABASE EMBL [Online] 1 November 1996 (1996-11-01), "YPR140W" XP002251185 Database accession no. Q06510
- DATABASE EMBL [Online] 16 June 2001 (2001-06-16), "Arabidopsis thaliana unknown protein" XP002251186 Database accession no. AF386968
- DATABASE EMBL [Online] 31 July 2001 (2001-07-31), "Arabidopsis thaliana unknown protein" XP002251187 Database accession no. AY045874

## Description

The invention relates to the use of genes that when expressed will increase the total amount of oil (i.e. triacylglycerols, monoacylglycerols, fatty acids) that is produced in transgenic organisms.

More specifically this invention describes the identification of genes encoding oil synthesis enhancing proteins (OEP).

In a first embodiment, this invention is directed to the use of a protein comprising an amino acid sequence as set forth in SEQ ID NO: 2 or a functional fragment, derivative, variant, or ortologue thereof.

The present invention further includes the use of a nucleotide sequence as set forth in SEQ ID NO: 1, as well as portions of the genomic sequence, the cDNA sequence, allelic variants, synthetic variants and mutants thereof. This includes sequences that are to be used as probes, vectors for transformation or cloning intermediates.

SEQ ID NO : 2 is the deduced amino acid sequence from the open reading frame YPR140w in SEQ ID NO : 1.

In a second embodiment, this invention is directed to the use of a protein comprising an amino acid sequence as set forth in SEQ ID NO: 4 or a functional fragment, derivative, variant, or ortologue thereof.

SEQ ID NO : 4 is the deduced amino acid sequence from the open reading frame At140.1 in SEQ ID NO : 3.

In a third embodiment, this invention is directed to the use of a protein comprising an amino acid sequence as set forth in SEQ ID NO: 6 or a functional fragment, derivative, variant, or ortologue thereof.

SEQ ID NO : 6 is the deduced amino acid sequence from the open reading frame At140.2 in SEQ ID NO : 5.

Another aspect of the present invention relates to the use of those polypeptides, which have at least 60% identity to SEQ ID NO: 2, SEQ ID NO : 4 or SEQ ID NO : 6.

The invention furthermore relates to expression constructs for expressing, At140.1 or At140.2 in plants, preferably in plant seeds, transgenic plants expressing YPR140w, At140.1 or At140.2 and to the use of said transgenic plants for the production of food, feed, seed, pharmaceuticals or fine chemicals, in particular for the production of oils.

In oil crops like rape, sunflower, oil palm etc., the oil (i.e. triacylglycerols) is the most valuable product of the seeds or fruits and other compounds such as starch, protein and fiber is regarded as by-products with less value. Enhancing the quantity of oil per weight basis at the expense of other compounds in oil crops would therefore increase the value of the crop. If proteins that promote the allocation of reduced carbon into the production of oil can be up regulated by overexpression, the cells will accumulate more oil at the expense of other products. This approach could not only be used to increase the oil content in already high oil producing organisms such as oil crops, they could also lead to significant oil production in moderate or low oil containing crops such as soy, oat, maize, potato, sugar beats, and turnips as well as in microorganisms.

Increasing the oil content in plants and, in particular, in plant seeds is of great interest for traditional and modem plant breeding and in particular for plant biotechnology. Owing to the increasing consumption of vegetable oils for nutrition or industrial applications, possibilities of increasing or modifying vegetable oils are increasingly the subject of current research (for example Töpfer et al. (1995) Science 268:681-686). Its aim is in particular increasing the fatty acid content in seed oils.

The fatty acids which can be obtained from the vegetable oils are also of particular interest. They are employed, for example, as bases for plasticizers, iubricants, surfactants, cosmetics and the like and are employed as valuable bases in the food and feed industries. Thus, for example, it is of particular interest to provide rapeseed oils with fatty acids with medium chain length since these are in demand in particular in the production of surfactants. With regard to medical ramifications, the long chain fatty acids (C18 and longer) found in many seed oils have been linked to reductions in hypercholesterolemia and other clinical disorders related to coronary heart disease (Brenner 1976, Adv. Exp. Med. Biol. 83:85-101). Therefore, consumption of a plant having increased levels of these types of fatty acids may reduce the risk of heart disease. Enhanced levels of seed oil content also increase large-scale production of seed oils and thereby reduce the cost of these oils.

The targeted modulation of plant metabolic pathways by recombinant methods allows the modification of the plant metabolism in an advantageous manner which, when using traditional breeding methods, could only be achieved after a complicated procedure or not at all. Thus, unusual fatty acids, for example specific polyunsaturated fatty acids, are only synthesized in certain plants or not at all in plants and can therefore only be produced by expressing the relevant gene in transgenic plants (for example Millar et al. (2000) Trends Plant Sci 5:95-101).

Triacylgylcerides, diacylglycerides, monoacylglycerides and other lipids are synthesized from fatty acids. Fatty acid biosynthesis and triacylglyceride biosynthesis can be considered as separate biosynthetic pathways owing to the compartmentalization, but as a single biosynthetic pathway in view of the end product. Lipid synthesis can be divided into two part-mechanisms, one which might be termed "prokaryotic" and another which may be termed "eukaryotic" (Browse et al. (1986) Biochemical J 235:25-31; Ohlrogge & Browse (1995) Plant Cell 7:957-970). The prokaryotic mechanism is localized in the plastids and encompasses the biosynthesis of the free fatty acids which are exported into the cytosol, where they enter the eukaryotic mechanism in the form of fatty acid acyl-CoA esters. In this pathway the fatty acids are esterified by glycerol-3-phosphate acyltransferase (GPAT) and lysophosphatidic acid acyltransferase to the sn-1 and sn-2 positions of glycerol-3-phosphate, respectively, to yield phosphatidic acid (PA). The PA is the precursor for other polar and neutral lipids, the latter being formed in the Kennedy pathway (Voelker 1996, Genetic Engineering ed.:Setlow 18:111-113; Shanklin & Cahoon 1998, Annu. Rev. Planet Physiol. Plant Mol. Biol. 49:611-641; Frentzen 1998, Lipids 100:161-166; Millar et al. 2000, Trends Plant Sci. 5:95-101).

Patent WO 00/18889 describes nucleic acid sequences encoding for acyltransferase related proteins, wherein said acyltransferase-like protein is active in the transfer of a fatty acyl group from a fatty acyl donor to fatty acyl acceptor and the use of such sequences to provide transgenic plants capable of producing modified lipid content and composition based on sequence predictions only. Biochemical data supporting these claims is lacking so far.

The initial step of lipid production, the transfer of a fatty acid from an acyl-CoA to glycerol-3-phosphate was shown to be in part but not exclusively performed by two yeast genes, Gat1 and Gat2 (Zeng andf Zou 2001, J Biol Chem. 276(45):41710-6). Patent WO 02/08391 relates to DNA sequences encoding these two novel glycerol-3-phosphate and dihydroxy acetone phosphate acyltransferase enzymes and methods for utilizing said polynucleotides for alteration of lipid content in higher cells, in particular plant cells. However, no counterparts from plants could be found for Gat1 or Gat2.

The last step in the synthesis of triacylglycerols has been shown to occur by two different enzymatic reactions, an acyl-CoA dependent reaction catalyzed by an acyl-CoA : diacylglycerol acyltransferase (Cases, S. et al., (1998) Proc. Natl. Acad. Sci., USA 95, 13018-13023.; Lardizabal, et al., 2001) and the acyl-CoA independent reaction catalyzed by an phospholipid : diacylglyerol acyltransferase (Dahlqvist, et al., 2000). Two unrelated gene families encoding acyl-CoA : diacylglycerol acyltransferases have been identified in plants, animals and yeast, whereas the gene family encoding the acyl-CoA independent enzyme has been identified in yeast but not in plants or animals. In yeast, a total of four genes (are1, are2, iro1, dga1) belong to these three gene families, and they are the only genes known to contribute directly to triacylglycerol synthesis. Thus, no synthesis of triacylglycerol could be detected in yeast cells where all four genes were disrupted.

It is an object of the present invention to provide additional methods for increasing the oil content in plants.

We have found that this object is achieved by the present invention.

In the present invention we show, that another gene is present in yeast and moreover in plants, which enhances the amount of triacylglycerol and fatty acids that accumulates in wild type yeast.

A first subject matter of the invention comprises a method of increasing the total oil content in a plant organism or a tissue, organ, part, cell or propagation material thereof, comprising
a) the transgenic expression of SEQ ID NO : 1, SEQ ID NO : 3 or SEQ ID NO : 5 in said plant organism or in a tissue, organ, part, cell or propagation material thereof, and
b) the selection of plant organisms in which - in contrast to or comparison with the starting organism - the total oil content in said plant organism or in a tissue, organ, part, cell or propagation material thereof is increased.

Other proteins resulting in the same effect as the proteins set forth in SEQ ID NO : 2, SEQ ID NO : 4 or SEQ ID NO : 6 (OEPs) are obtainable from the specific sequences provided herein. Furthermore, it will be apparent that one can obtain natural and synthetic OEPs, including those with modified amino acid sequences and starting materials for synthetic-protein modeling from the exemplified OEPs and from OEPs which are obtained through the use of such exemplified sequences. Modified amino acid sequences include sequences that have been mutated, truncated, increased and the like, whether such sequences were partially or wholly synthesized.

Further, the nucleic acid probes (DNA or RNA) derived from the SEQ ID NO : 1, SEQ ID NO : 3 or SEQ ID NO: 5 of the present invention can be used to screen and recover homologous or related sequences from a variety of plant and microbial sources.

The over-expression of SEQ ID NO : 3 or SEQ ID NO : 5 in yeast or plants increases the content in total lipids and enhances the amount of triacylglycerol and fatty acids that accumulates compared to wildtype yeasts or plants. The amount of polar lipids remained unchanged but a reduced amount of palmitic acid was observed. On the other hand the amount of oleic acids was increased.

The present invention can be characterized by the following aspects:
Example 1
   shows the reduction of GPAT activity in yeast cells lacking the YPR140w gene.
Example 2
   shows the increased incorporation of glycerol-3-phosphate into lipids in crude extracts of yeast over expressing the YPR140w gene.
Example 3
   shows the increased accumulation of triacylglycerol in yeast cells expressing the YPR140w gene from a strong promoter.
Example 4
   describes the identification of genes homologous to YPR140w in plants.
Example 5
   shows the increased accumulation of triacylglycerol in yeast cells expressing the At140.1 or At140.2 gene from a strong promoter.
Example 6
   shows a significantly higher total oil content in the seeds of transgenic plant lines with increased expression of the At140.1, At140.2 or YPR140w gene.

The invention can furthermore be characterized by:
use of the nucleic acid sequences SEQ ID NO : 1, SEQ ID NO : 3 or SEQ ID NO : 5 encoding a protein SEQ ID NO : 2, SEQ ID NO : 4 or SEQ ID NO : 6 that enhances the production of triacylglycerol (TAG);
genetic transformation of an oil-producing organism with one of said sequence in order to be expressed in this organism, resulting in an active protein that increases the oil content of the organism.

The nucleic acid sequence is derived from the sequence shown in SEQ ID NO : 1 from the Saccharomyces cerevisiae YPR140w gene (genomic clone or cDNA). SEQ ID NO: 3 or SEQ ID NO: 5 from Arabidopsis thaliana or from a nucleic acid sequence or cDNA that contains a nucleotide sequence coding for a protein with an amino acid sequence that is 60% or more identical to the amino acid sequences as presented in SEQ ID NO: 2, SEQ ID NO: 4 or SEQ ID NO: 6.

The gene products, which we refer to as an oil synthesis enhancing protein (OEP) is most likely not itself catalyzing the synthesis of TAG, but its presence elevates the amount of TAG synthesized.

Furthermore the over expression of the OEP as characterised by the protein sequence SEQ ID NO : 2, SEQ ID NO : 4 or SEQ ID NO : 6 results in an increased incorporation of (¹⁴C) glycerol-3-phosphate into lipids as described in example 3, 5 and 6.

The instant invention pertains to a gene construct comprising a said nucleotide sequence SEQ ID NO: 1, SEQ ID NO: 3 or SEQ ID NO: 5 of the instant invention, which is operably linked to a heterologous nucleic acid.

The term operably linked means a serial organization e.g. of a promoter, coding sequence, terminator and/or further regulatory elements whereby each element can fulfill its original function during expression of the nucleotide sequence.

Further, a vector comprising the said nucleotide sequence SEQ ID NO: 3 or SEQ ID NO: 5 of the instant invention is contemplated in the instant invention. This includes also an expression vector which can harbor a selectable marker gene and/or nucleotide sequences for the replication in a host cell and/or the integration into the genome of the host cell.

Furthermore, this invention relates to a method for producing an OEP encoded by one of the nuleic acid sequences SEQ ID NO: 1. SEQ ID NO: 3 or SEQ ID NO: 5 in a host cell or progeny thereof including genetically engineered oil seeds, yeast and moulds or any other oil-accumulating organism, via the expression of a construct in the cell. Of particular interest is the expression of the nucleotide sequences of the present invention from transcription initiation regions that are preferentially expressed in plant seed tissues. It is further contemplated that an artificial gene sequence encoding an OEP may be synthesized, especially to provide plant-preferred codons. Transgenic cells containing an OEP as a result of the expression of an OEP encoding sequence are also contemplated within the scope of the invention.

Further, the invention pertains a transgenic cell or organism containing a said nucleotide sequence and/or a said gene construct and/or a said vector. The object of the instant invention is further a transgenic cell or organism which is an eucaryotic cell or organism. Preferably, the transgenic cell or organism is a yeast cell or a plant cell or a plant. The instant invention further pertains said transgenic cell or organism having an increased biosynthetic pathway for the production of substrates for the synthesis of triacylglycerol. A transgenic cell or organism having increased oil content is also contemplated within the scope of this invention.

Further, the invention pertains a transgenic cell or organism wherein the activity of an OEP encoded by one of the nuleic acid sequences SEQ ID NO: 3 or SEQ ID NO: 5 is increased in said cell or organism. The increased activity of OEP is characterized by an alteration in gene expression, catalytic activity and/or regulation of activity of the enzyme. Moreover, a transgenic cell or organism is included in the instant invention, wherein the increased biosynthetic pathway for the production of substrates for the production of triacylglycerol is characterized e.g. by the prevention of accumulation of undesirable fatty acids in the membrane lipids.

In a different embodiment, this invention also relates to methods of using a DNA sequence SEQ ID NO: 1, SEQ ID NO: 3 or SEQ ID NO: 5 for increasing the oil-content within the cells of different organisms.

Further, the invention makes possible a process for elevating the production of triacylglycerol, which comprises growing transgenic cells or organisms under conditions whereby the nucleotide sequences SEQ ID NO: 1, SEQ ID NO : 3 or SEQ ID NO : 5 are expressed in order to produce a protein in these cells with the ability of enhancing the production of triacylglycerol.

Corresponding genes coding for OEP can be isolated from other organisms, especially yeast-type organisms, like e.g. Schizosaccharomyces pombe, Yarrowia lipolytica, Zygosaccharomyces rouxii, Saccharomyces cerevisiae, Emericella nidulans and Debaryomyces hansenii.

Furthermore the invention pertains transgenic organisms comprising, in their genome or on a plasmid, a nucleic acid sequence SEQ ID NO : 3 or SEQ ID NO: 5 according to the above, transferred by recombinant DNA technology. One important type of transgenic organism covered by this invention are commercially relevant plants in which said nucleotide sequence preferably would be expressed under the control of a storage organ specific promoter. Alternatively, the nucleotide sequence could also be expressed under the control of a seed-specific promoter or any other promoter suitable for tissue-specific high-level expression in plants.

The invention also pertains a protein encoded by a DNA molecule according to SEQ ID NO: 3 or SEQ ID NO: 5 or a functional biologically active fragment thereof having OEP activity in transgenic organisms. Alternatively, the invention pertains a protein produced in an organism, which has the amino acid sequence set forth in SEQ ID NO: 4 or SEQ ID NO: 6 or an amino acid sequence with at least 60% homology to said amino acid sequence having OEP activity.

The protein can be is isolated from Saccharomyces cerevisiae or Arabidopsis thaliana but also from other organisms.

The invention additionally pertains the use of a protein according to SEQ ID NO: 2, SEQ ID NO: 4 or SEQ ID NO: 6 or derivatives of that protein having OEP activity for the increased production of triacylglycerols, monoacylglycerol and/or fatty acids.

Surprisingly, it has been found that the homologous expression of the corresponding genes of Arabidopsis thaliana SEQ ID NO: 3 or SEQ ID NO: 5 in Arabidopsis leads to a significantly increased triacylglyceride (storage oils) content in the seeds of T3 plants as described in example 6. The oil content was increased by more than 5%, preferably by 10%, most preferably by 15% compared with wild-type control plants. The over-expression of the At140.1 or At140.2 genes had no adverse effects on the growth or other properties of the transformed plants.

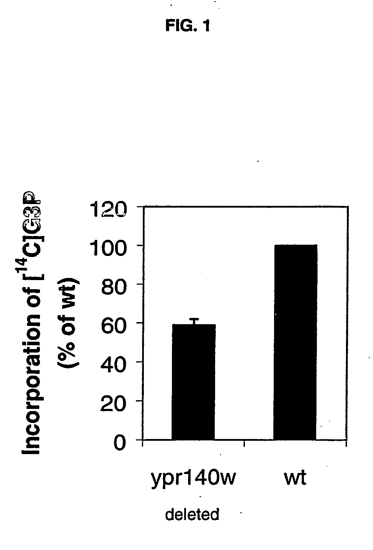

Figure 1 shows the effect of deleting the YPR140w gene in yeast on in vitro lipid biosynthesis. The incorporation rate of [¹⁴C]glycerol-3phosphate ([¹⁴C]G3P) into lipids was analyzed in microsomal fractions of YPR140w-deletion and wt yeast (Saccharomyces cerevisiae) strains. Yeast strains were grown in rich media to an optical density of 1.5 (A₆₀₀) and microsomal fractions were prepared. Enzymatic assays (n=4) were performed for 10 minutes at 30°C as described in Example 1.

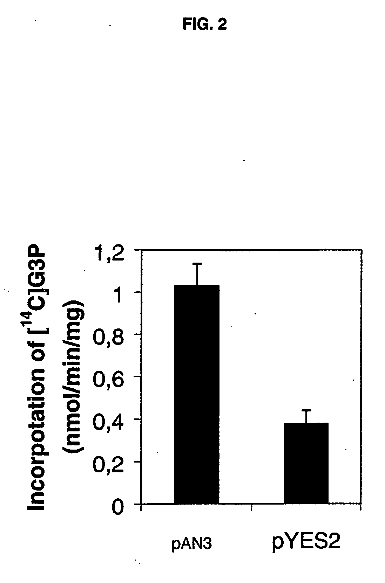

The effect of over expressing gene YPR140w in yeast on the in vitro incorporation rate of [¹⁴C]G3P into lipids is demonstrated in figure 2. The analyses were performed with crude extracts of Saccharomyces cerevisiae transformed with pAN3, multicopy plasmid with the gene YPR140w behind the GAL1 promotor, or pYES2, empty plasmid control as described in Example 2. Data presented are average from 8 replicates.

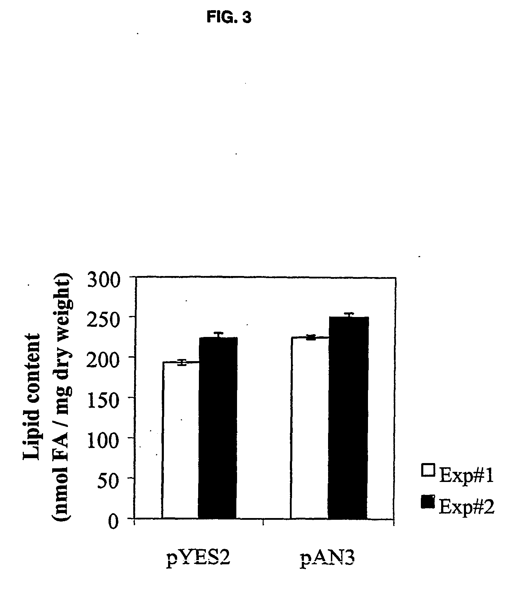

In figure 3 the total lipid content in the YPR140w yeast transformant harvested 24 hours (Exp.#1; open bar) or 28 hours (Exp.#2; filled bar) after induction is shown. Yeast cells were transformed with the pAN3 vector expressing the YPR140w from the GAL1 inducible promotor. Yeast cells transformed with empty vector (pYES2) were used as control. Total lipid content was determined as nmol fatty acid (FA) per mg dry cell weight.

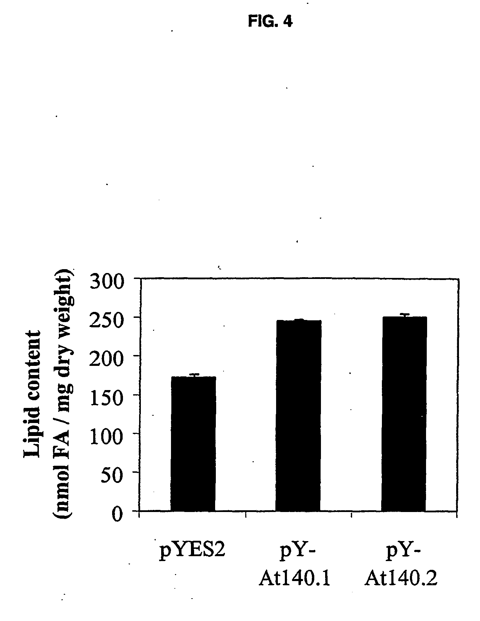

In figure 4 the total lipid content in the different yeast transformants harvested after 24 hours is shown. Yeast cells were transformed with the pY-At140.1 or the pY-At140.2 vector expressing the At140.1 or At140.2 from the GAL1 inducible promotor. Yeast cells transformed with empty vector (pYES2) were used as control. Total lipid content was determined as nmol fatty acid (FA) per mg dry cell weight.

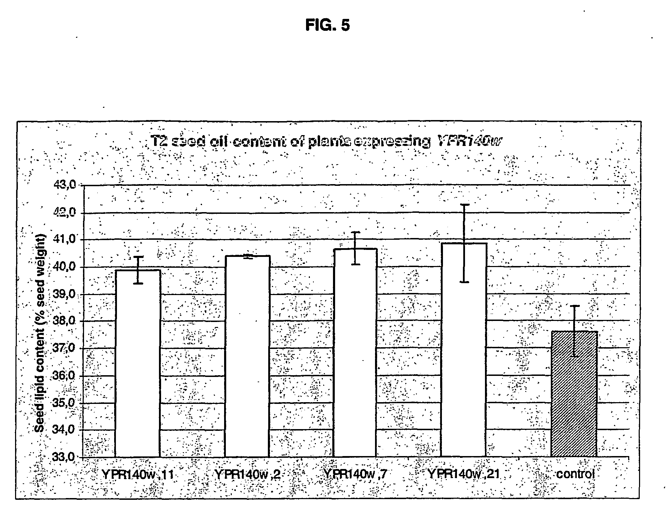

In figure 5 the total lipid content of dry T2 generation seeds from Arabidopsis plants overexpressing At140.1 and At140.2 is shown. Wildtype Arabidopsis plants were used as control. Total lipid content was determined by conventional gas chromatography as mg FA per mg dry seeds.

The method according to the invention can be applied in principle to all plant species, in addition to the species Arabidopsis thaliana, which is employed as model plant. The method according to the invention is preferably applied to oil crops whose oil content is already naturally high and/or for the industrial production of oils.

Plant organism or tissue, organ, part, cell or propagation material thereof is generally understood as meaning any single- or multi-celled organism or a cell, tissue, part or propagation material (such as seeds or fruit) of same which is capable of photosynthesis. Included for the purpose of the invention are all genera and species of higher and lower plants of the Plant Kingdom. Annual, perennial, monocotyledonous and dicotyledonous plants are preferred. Also included are mature plants, seeds, shoots and seedlings, and parts, propagation material (for example tubers, seeds or fruits) and cultures derived from them, for example cell cultures or callus cultures.

Plant encompasses all annual and perennial monocotyledonous or dicotyledonous plants and includes by way of example, but not by limitation, those of the genera Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Cichorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea and Populus.

Preferred plants are those from the following plant families: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanaceae, Sterculiaceae, Tetragoniaceae, Theaceae, Umbelliferae.

Preferred monocotyledonous plants are selected in particular from the monocotyledonous crop plants such as, for example, the Gramineae family, such as rice, maize, wheat or other cereal species such as barley, millet and sorghum, rye, triticale or oats, and sugar cane, and all grass species.

The invention is applied very particularly preferably to dicotyledonous plant organisms. Preferred dicotyledonous plants are selected in particular from the dicotyledonous crop plants such as, for example,
- Asteraceae such as Heliantus annuus (sunflower), tagetes or calendula and others,
- Compositae, especially the genus Lactuca, very particularly the species sativa (lettuce) and others,
- Cruciferae, particularly the genus Brassica, very particularly the species napus (oilseed rape), campestris (beet), oleracea cv Tastie (cabbage), oleracea cv Snowball Y (cauliflower) and oleracea cv Emperor (broccoli) and other cabbages; and the genus Arabidopsis, very particularly the species thaliana, and cress or canola and others,
- Cucurbitaceae such as melon, pumpkin/squash or zucchini and others,
- Leguminosae, particularly the genus Glycine, very particularly the species max (soybean), soya, and alfalfa, pea, beans or peanut and others,
- Rubiaceae, preferably the subclass Lamiidae such as, for example Coffea arabica or Coffea liberica (coffee bush) and others,
- Solanaceae, particularly the genus Lycopersicon, very particularly the species esculentum (tomato), the genus Solanum, very particularly the species tuberosum (potato) and melongena (aubergine) and the genus Capsicum, very particularly the genus annuum (pepper) and tobacco or paprika and others,
- Sterculiaceae, preferably the subclass Dilleniidae such as, for example, Theobroma cacao (cacao bush) and others,
- Theaceae, preferably the subclass Dilleniidae such as, for example, Camellia sinensis or Thea sinensis (tea shrub) and others,
- Umbelliferae, particularly the genus Daucus (very particularly the species carota (carrot)) and Apium (very particularly the species graveolens dulce (celeary)) and others;
and linseed, cotton, hemp, flax, cucumber, spinach, carrot, sugar beet and the various tree, nut and grapevine species, in particular banana and kiwi fruit.

Also encompassed are ornamental plants, useful or ornamental trees, flowers, cut flowers, shrubs or turf plants which may be mentioned by way of example but not by limitation are angiosperms, bryophytes such as, for example, Hepaticae (liverworts) and Musci (mosses); pteridophytes such as ferns, horsetail and clubmosses; gymnosperms such as conifers, cycades, ginkgo and Gnetatae; algae such as Chlorophyceae, Phaeophpyceae, Rhodophyceae, Myxophyceae, Xanthophyceae, Bacillariophyceae (diatoms) and Euglenophyceae. Plants within the scope of the invention comprise by way of example and not by way of limitation, the families of the Rosaceae such as rose, Ericaceae such as rhododendron and azalea, Euphorbiaceae such as poinsettias and croton, Caryophyllaceae such as pinks, Solanaceae such as petunias, Gesneriaceae such as African violet, Balsaminaceae such as touch-me-not, Orchidaceae such as orchids, Iridaceae such as gladioli, iris, freesia and crocus, Compositae such as marigold, Geraniaceae such as geranium, Liliaceae such as dracena, Moraceae such as ficus, Araceae such as cheeseplant and many others.

Furthermore, plant organisms for the purposes of the invention are further organisms capable of being photosynthetically active such as, for example, algae, cyanobacteria and mosses. Preferred algae are green algae such as, for example, algae from the genus Haematococcus, Phaedactylum tricomatum, Volvox or Dunaliella. Synechocystis is particularly preferred.

Most preferred are oil crops. Oil crops are understood as being plants whose oil content is already naturally high and/or which can be used for the industrial production of oils. These plants can have a high oil content and/or else a particular fatty acid composition which is of interest industrially. Preferred plants are those with a lipid content of at least 1% by weight. Oil crops encompassed by way of example: Borvago officinalis (borage); Brassica species such as *B. campestris, B. napus, B. rapa* (mustard, oilseed rape or turnip rape); *Cannabis sativa* (hemp); Carthamus *tinctorius* (safflower); Cocos *nucifera* (coconut); Crambe abyssinica (crambe); Cuphea species (Cuphea species yield fatty acids of medium chain length, in particular for industrial application); *Elaeis guinensis* (African oil palm); *Elaeis oleifera* (American oil palm); *Glycine max* (soybean); *Gossypium hirisutfum* (American cotton); *Gossypium barbadense* (Egyptian cotton); *Gossypium herbaceum* (Asian cotton); *Helianthus annuus* (sunflower); *Linum usitatissimum* (linseed or flax); *Oenothera biennis* (evening primrose); *Olea europaea* (olive); Oryza *sativa* (rice); *Ricinus communis* (castor); Sesamum *indicum* (sesame); *Triticum* species (wheat); Zea *mays* (maize), and various nut species such as, for example, walnut or almond.

"Total oil content" refers to the sum of all oils, preferably to the sum of the triacylglycerides.

"Oils" encompasses neutral and/or polar lipids and mixtures of these. Those mentioned in Table 1 may be mentioned by way of example, but not by limitation.

**Table 1: Classes of plant lipids**

| | |
|---|---|
| Neutrale lipids | Triacylglycerol (TAG) |
| | Diacylglycerol (DAG) |
| | Monoacylglycerol (MAG) |
| | |
| Polar lipids | Monogalactosyldiacylglycerol (MGDG) |
| | Digalactosyldiacylglycerol (DGDG) |
| | Phosphatidylglycerol (PG) |
| | Phosphatidylcholine (PC) |
| | Phosphatidylethanolamine (PE) |
| | Phosphatidylinositol (PI) |
| | Phosphatidylserine (PS) |
| | Sulfoquinovosyldiacylglycerol |

Neutral lipids preferably refers to triacylglycerides. Both neutral and polar lipids may comprise a wide range of various fatty acids. The fatty acids mentioned in Table 2 may be mentioned by way of example, but not by limitation.

**Table 2: Overview over various fatty acids (selection)**

| Nomenclature¹ | Name |
|---|---|
| 16:0 | Palmitic acid |
| 16:1 | Palmitoleic acid |
| 16:3 | Roughanic acid |
| 18:0 | Stearic acid |
| 18:1 | Oleic acid |
| 18:2 | Linoleic acid |
| 18:3 | Linolenic acid |
| γ-18:3 | Gamma-linolenic acid * |
| 20:0 | Arachidic acid |
| 22:6 | Docosahexaenoic acid (DHA) * |
| 20:2 | Eicosadienoic acid |
| 20:4 | Arachidonic acid (AA) * |
| 20:5 | Eicosapentaenoic acid (EPA) * |
| 22:1 | Erucic acid |

| | |
|---|---|
| ¹ Chain length: number of double bonds * not naturally occurring in plants | |

Oils preferably relates to seed oils.

"Increase in" the total oil content refers to the increased oil content in a plant or a part, tissue or organ thereof, preferably in the seed organs of the plants. In this context, the oil content is at least 5%, preferably at least 10%, particularly preferably at least 15%, very particularly preferably at least 20%, most preferably at least 25% increased under otherwise identical conditions in comparison with a starting plant which has not been subjected to the method according to the invention, but is otherwise unmodified. Conditions in this context means all of the conditions which are relevant for germination, culture or growth of the plant, such as soil conditions, climatic conditions, light conditions, fertilization, irrigation, plant protection treatment and the like.

"YPR140w" generally refers to all those proteins which are capable of increasing the oil content in oil producing organims, especially microorganisms, yeast, fungi and plants and are identical to SEQ ID NO: 2 or have homology to SEQ ID NO: 2.

Yeast refers to the group of unicellular fungi with a pronounced cell wall and formation of pseudomycelium (in contrast to molds). They reproduce vegetatively by budding and/or fission (Schizosaccharomyces and Saccharomycodes, respectively). Encompassed are what are known as false yeasts, preferably the families Cryptococcaceae, Sporobolomycetaceae with the genera Cryptococcus, Torulopsis, Pityrosporum, Brettanomyces, Candida, Kloeckera, Trigonopsis, Trichosporon, Rhodotorula and Sporobolomyces and Bullera, and true yeasts (yeasts which also reproduce sexually; ascus), preferably the families Endo- and Saccharomycetaceae, with the genera Saccharomyces, Debaromyces, Lipomyces, Hansenula, Endomycopsis, Pichia, Hanseniaspora. Most preferred are the genera Saccharomyces cerevisiae, Pichia pastoris, Hansenula polymorpha, Schizosaccharomyces pombe, Kluyveromyces lactis, Zygosaccharomyces rouxii, und Yarrowia lipolitica, Emericella nidulans, Aspergillus nidulans, Debaryomyces hansenii and Torulaspora hansenii.

YPR140w refers in particular to the polypeptide sequence SEQ ID NO: 2.

Most preferably, YPR140w refers to the yeast protein OEP as shown in SEQ ID NO : 2 and functional equivalents or else functionally equivalent portions of the above.

"At140.1" and "At140.2" refer in particular to the polypeptide sequence SEQ ID NO : 4 and SEQ ID NO : 6.

Most preferably, At140.1 and At140.2 refer to the *Arabidopsis thaliana* protein OEP as shown in SEQ ID NO: 4 and SEQ ID NO: 6 and functional equivalents or else functionally equivalent portions of the above.

Functional equivalents refers in particular to natural or artificial mutations of the yeast protein OEP as shown in SEQ ID NO: 2 and homologous polypeptides from other yeasts which have the same essential characteristics of YPR140w as defined above. Mutations encompass substitutions, additions, deletions, inversions or insertions of one or more amino acid residues.

The YPR140w to be employed advantageously within the scope of the present invention can be found readily by database searches or by screening gene or cDNA libraries using the polypeptide sequence shown in SEQ ID NO: 2, which is given by way of example, or the nucleic acid sequence as shown in SEQ ID NO: 1, which encodes the latter, as search sequence or probe.

Said functional equivalents preferably have at least 60%, particularly preferably at least 70%, particularly preferably at least 80%, most preferably at least 90% homology with the protein of SEQ ID NO: 2.

Functional equivalents refer in particular to natural or artificial mutations of the *Arabidopsis thaliana* OEP as shown in SEQ ID NO: 4 or SEQ ID NO: 6 and homologous polypeptides from other plants which have the same essential characteristics of an OEP as defined above. Mutations encompass substitutions, additions, deletions, inversions or insertions of one or more amino acid residues.

The nucleic acid to be employed advantageously within the scope of the present invention can be found readily by database searches or by screening gene or cDNA libraries using the polypeptide sequences shown in SEQ ID NO: 4 or SEQ ID NO: 6, which is given by way of example, or the nucleic acid sequence as shown in SEQ ID NO: 3 and SEQ ID NO: 5, which encode the latter, as search sequence or probe.

Furthermore the following nucleic acid sequences can be used in order to identify and clone genes encoding an OEP from plant organisms and having at least 60% homology to SEQ ID NO: 3 or SEQ ID NO: 5:
Arabidopsis thaliana ESTs from GenBank: AV782272, AV821735, AV825509, AI996105, AV791433, BE528444
Glycine max ESTs from GenBank: AW458589, BQ740950, BI893252, BG154651, AW507932
Rose Petals EST from GenBank: BQ105746
Rice EST from GenBank: AU166240
Tomato ESTs from GenBank: BG123776, AW218643
Medicago truncatula ESTs from GenBank: BG644974, CA858351
Gossypium arboreum EST from GenBank: BQ403497
Potato EST from GenBank: BE342183

Furthermore the nucleic acid sequences SEQ ID NO: 7 to SEQ ID NO: 35 can be used in order to identify and clone genes encoding an OEP from plant organisms having at least 60% homology to SEQ ID NO: 3 or SEQ ID NO: 5.

Said functional equivalents have at least 60%, preferably at least 70%, particularly preferably at least 80%, most preferably at least 90% homology with the protein of SEQ ID NO: 4 or SEQ ID NO: 6.

The functional equivalent of SEQ ID NO: 2, SEQ ID NO: 4 or SEQ ID NO: 6 has an identity of at least 50%, 51%, 52%, 53%, 54%, 55%, 56%, 57% preferably at least 58%, 59%, 60%, 61%, 62%, 63%, 64%, 65%, 66%, 67%, 68%, 69%, and 70% more preferably 71%, 72%, 73%, 74%, 75%, 76%, 77%, 78%, 79%, 80%, 81%, 82%, 83%, 84%, 85% most preferably at least 86%, 87%, 88%, 89%, 90%, 91%, 92%, 93%, 94%, 95%, 96%, 97%, 98%, 99% identity with the SEQ ID NO: 2, SEQ ID NO: 4 or SEQ ID NO: 6.

Homology between two polypeptides is understood as meaning the identity of the amino acid sequence over the entire sequence length which is calculated by comparison with the aid of the program algorithm GAP (Wisconsin Package Version 10.0, University of Wisconsin, Genetics Computer Group (GCG), Madison, USA), setting the following parameters:

| | |
|---|---|
| Gap Weight: 8 | Length Weight: 2 |
| | |
| Average Match: 2,912 | Average Mismatch: -2,003 |

For example, a sequence with at least 80% homology with the sequence SEQ ID NO: 2 at the protein level is understood as meaning a sequence which, upon comparison with the sequence SEQ ID NO: 2 with the above program algorithm and the above parameter set has at least 80% homology.

Functional equivalents - for example - also encompass those proteins which are encoded by nucleic acid sequences which have at least 60%, particularly preferably at least 70%, particularly preferably at least 80%, most preferably at least 90% homology with the nucleic acid sequence with the SEQ ID NO: 1.

Homology between two nucleic acid sequences is understood as meaning the identity of the two nucleic acid sequences over the entire sequence length which is calculated by comparison with the aid of the program algorithm GAP (Wisconsin Package Version 10.0, University of Wisconsin, Genetics Computer Group (GCG), Madison, USA), setting the following parameters:

| | |
|---|---|
| Gap Weight: 50 | Length Weight: 3 |
| | |
| Average Match: 10 | Average Mismatch:0 |

For example, a sequence which has at least 80% homology with the sequence SEQ ID NO: 1 at the nucleic acid level is understood as meaning a sequence which, upon comparison with the sequence SEQ ID NO: 1 within the above program algorithm with the above parameter set has a homology of at least 80%.

Functional equivalents also encompass those proteins which are encoded by nucleic acid sequences which hybridize under standard conditions with a nucleic acid sequence described by SEQ ID NO: 1, SEQ ID NO: 3 or SEQ ID NO: 5, the nucleic acid sequence which is complementary thereto or parts of the above and which have the essential characteristics of an OEP as characterised by SEQ ID NO : 2, SEQ ID NO: 4 or SEQ ID NO: 6.

Natural examples of OEPs and the corresponding genes can furthermore readily be found in various organisms, in particular plants, whose genomic sequence is unknown, by hybridization techniques in a manner known per se, for example starting from the nucleic acid sequences SEQ ID NO : 1, SEQ ID NO : 3 or SEQ ID NO : 5.

The hybridization may be carried out under moderate (low stringency) or, preferably, under stringent (high stringency) conditions.

Such hybridization conditions are described, inter alia, in Sambrook, J., Fritsch, E.F., Maniatis, T., in: Molecular Cloning (A Laboratory Manual), 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

By way of example, the conditions during the washing step may be selected from the range of conditions which is limited by those with low stringency (with 2X SSC at 50°C) and those with high stringency (with 0.2X SSC at 50°C, preferably at 65°C) (20X SSC: 0.3 M sodium citrate, 3 M sodium chloride, pH 7.0).

In addition, the temperature may be raised during the washing step from moderate conditions at room temperature, 22°C, to stringent conditions at 65°C.

Both parameters, salt concentration and temperature, may be varied simultaneously and it is also possible to keep one of the two parameters constant and to vary only the other one. It is also possible to use denaturing agents such as, for example, formamide or SDS during hybridization. In the presence of 50% formamide, the hybridization is preferably carried out at 42°C.

Some exemplary conditions for hybridization and washing step are listed below:
(1.) hybridization conditions with, for example
   (i) 4X SSC at 65°C, or
   (ii) 6X SSC at 45°C, or
   (iii) 6X SSC at 68°C, 100 mg/ml denatured fish sperm DNA, or
   (iv) 6X SSC, 0.5% SDS, 100 mg/ml denatured fragmented salmon sperm DNA at 68°C, or
   (v) 6X SSC, 0.5% SDS, 100 mg/ml denatured fragmented salmon sperm DNA, 50% formamide at 42°C, or
   (vi) 50% formamide, 4X SSC at 42°C, or
   (vii) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C, or
   (viii) 2X or 4X SSC at 50°C (moderate conditions), or
   (ix) 30 to 40% formamide, 2X or 4X SSC at 42°C (moderate conditions).
(2.) Washing steps of 10 minutes each with, for example
   (i) 0.015 M NaCl/0.0015 M sodium citrate/0.1% SDS at 50°C, or
   (ii) 0.1X SSC at 65°C, or
   (iii) 0.1X SSC, 0.5% SDS at 68°C, or
   (iv) 0.1X SSC, 0.5% SDS, 50% formamide at 42°C, or
   (v) 0.2X SSC, 0.1% SDS at 42°C, or
   (vi) 2X SSC at 65°C (moderate conditions).

The invention furthermore relates to transgenic expression constructs which can ensure a transgenic expression of an OEP as characterised by SEQ ID NO: 4 or SEQ ID NO: 6 in a plant organism or a tissue, organ, part, cells or propagation material of said plant organism.

The definition given above applies to an OEP, with the transgenic expression of an OEP described by the sequence with the SEQ ID NO: 4 or SEQ ID NO: 6 being particularly preferred.

In said transgenic expression constructs, a nucleic acid molecule encoding an OEP is preferably in operable linkage with at least one genetic control element (for example a promoter) which ensures expression in a plant organism or a tissue, organ, part, cell or propagation material of same.

Especially preferred are transgenic expression cassettes wherein the nucleic acid sequence encoding an OEP is described by
a) a sequence SEQ ID NO : 1, SEQ ID NO: 3 or SEQ ID NO: 5
b) a sequence derived from a sequence SEQ ID NO: 1, SEQ ID NO: 3 or SEQ ID NO: 5 in accordance with the degeneracy of the genetic code
c) a sequence which has at least 60% identity with a sequence SEQ ID NO: 1, SEQ ID NO: 3 or SEQ ID NO: 5.

Operable linkage is understood as meaning, for example, the sequential arrangement of a promoter with the nucleic acid sequence encoding an OEP which is to be expressed (for example the sequence as shown in SEQ ID NO: 1, SEQ ID NO: 3 or SEQ ID NO: 5 and, if appropriate, further regulatory elements such as, for example, a terminator in such a way that each of the regulatory elements can fulfil its function when the nucleic acid sequence is expressed recombinantly. Direct linkage in the chemical sense is not necessarily required for this purpose. Genetic control sequences such as, for example, enhancer sequences can also exert their function on the target sequence from positions which are further removed or indeed from other DNA molecules. Preferred arrangements are those in which the nucleic acid sequence to be expressed recombinantly is positioned behind the sequence acting as promoter so that the two sequences are linked covalently to each other. The distance between the promoter sequence and the nucleic acid sequence to be expressed recombinantly is preferably less than 200 base pairs, particularly preferably less than 100 base pairs, very particularly preferably less than 50 base pairs.

Operable linkage and a transgenic expression cassette can both be effected by meansof conventional recombination and cloning techniques as they are described, for example, in Maniatis T, Fritsch EF and Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience and in Gelvin et al. (1990) In: Plant Molecular Biology Manual. However, further sequences which, for example, act as a linker with specific cleavage sites for restriction enzymes, or of a signal peptide, may also be positioned between the two sequences. Also, the insertion of sequences may lead to the expression of fusion proteins. Preferably, the expression cassette composed of a promoter linked to a nucleic acid sequence to be expressed can be in a vector-integrated form and can be inserted into a plant genome, for example by transformation.

However, a transgenic expression cassette is also understood as meaning those constructs where the nucleic acid sequence encoding an OEP is placed behind an endogenous plant promoter in such a way that the latter brings about the expression of the OEP.

Promoters which are preferably introduced into the transgenic expression cassettes are those which are operable in a plant organism or a tissue, organ, part, cell or propagation material of same. Promoters which are operable in plant organisms is understood as meaning any promoter which is capable of governing the expression of genes, in particular foreign genes, in plants or plant parts, plant cells, plant tissues or plant cultures. In this context, expression may be, for example, constitutive, inducible or development-dependent.

The following are preferred:
a) Constitutive promoters
   "Constitutive" promoters refers to those promoters which ensure expression in a large number of, preferably all, tissues over a substantial period of plant development, preferably at all times during plant development (Benfey et al.(1989) EMBO J 8:2195-2202). A plant promoter or promoter originating from a plant virus is especially preferably used. The promoter of the CaMV (cauliflower mosaic virus) 35S transcript (Franck et al. (1980) Cell 21:285-294; Odell et al. (1985) Nature 313:810-812; Shewmaker et al. (1985) Virology 140:281-288; Gardner et al. (1986) Plant Mol Biol 6:221-228) or the 19S CaMV promoter (US 5,352,605; WO 84/02913; Benfey et al. (1989) EMBO J 8:2195-2202) are especially preferred. Another suitable constitutive promoter is the Rubisco small subunit (SSU) promoter (US 4,962,028), the leguminB promoter (GenBank Acc. No. X03677), the promoter of the nopalin synthase from Agrobacterium, the TR dual promoter, the OCS (octopine synthase) promoter from Agrobacterium, the ubiquitin promoter (Holtorf S et al. (1995) Plant Mol Biol 29:637-649), the ubiquitin 1 promoter (Christensen et al. (1992) Plant Mol Biol 18:675-689; Bruce et al. (1989) Proc Natl Acad Sci USA 86:9692-9696), the Smas promoter, the cinnamyl alcohol dehydrogenase promoter (US 5,683,439), the promoters of the vacuolar ATPase subunits, the promoter of the Arabidopsis thaliana nitrilase-1 gene (GenBank Acc. No.: U38846, nucleotides 3862 to 5325 or else 5342) or the promoter of a proline-rich protein from wheat (WO 91/13991), and further promoters of genes whose constitutive expression in plants is known to the skilled worker. The CaMV 35S promoter and the Arabidopsis thaliana nitrilase-1 promoter are particularly preferred.
b) Tissue-specific promoters
   Furthermore preferred are promoters with specificities for seeds, such as, for example, the phaseolin promoter (US 5,504,200; Bustos MM et al. (1989) Plant Cell 1(9):839-53), the promoter of the 2S albumin gene (Joseffson LG et al. (1987) J Biol Chem 262:12196- 12201), the legumine promoter (Shirsat A et al. (1989) Mol Gen Genet 215(2):326-331), the USP (unknown seed protein) promoter (Bäumlein H et al. (1991) Mol Gen Genet 225(3):459-67), the napin gene promoter (US 5,608,152; Stalberg K et al. (1996) L Planta 199:515-519), the promoter of the sucrose binding proteins (WO 00/26388) or the legumin B4 promoter (LeB4; Bäumlein H et al. (1991) Mol Gen Genet 225: 121-128; Bäumlein et al. (1992) Plant Journal 2(2):233-9; Fiedler U et al. (1995) Biotechnology (NY) 13(10):1090f), the Arabidopsis oleosin promoter (WO 98/45461), and the Brassica Bce4 promoter (WO 91/13980).
   Further suitable seed-specific promoters are those of the gene encoding high-molecular weight glutenin (HMWG), gliadin, branching enyzme, ADP glucose pyrophosphatase (AGPase) or starch synthase. Promoters which are furthermore preferred are those which permit a seed-specific expression in monocots such as maize, barley, wheat, rye, rice and the like. The promoter of the Ipt2 or Ipt1 gene (WO 95/15389, WO 95/23230) or the promoters described in WO 99/16890 (promoters of the hordein gene, the glutelin gene, the oryzin gene, the prolamin gene, the gliadin gene, the glutelin gene, the zein gene, the casirin gene or the secalin gene) can advantageously be employed.
c) Chemically inducible promoters
   The expression cassettes may also contain a chemically inducible promoter (review article: Gatz et al. (1997) Annu Rev Plant Physiol Plant Mol Biol 48:89-108), by means of which the expression of the exogenous gene in the plant can be controlled at a particular point in time. Such promoters such as, for example, the PRP1 promoter (Ward et al. (1993) Plant Mol Biol 22:361-366), a salicylic acid-inducible promoter (WO 95/19443), a benzenesulfonamide-inducible promoter (EP 0 388 186), a tetracyclin-inducible promoter (Gatz et al. (1992) Plant J 2:397-404), an abscisic acid-inducible promoter EP 0 335 528) or an ethanol-cyclohexanone-inducible promoter (WO 93/21334) can likewise be used. Also suitable is the promoter of the glutathione-S transferase isoform II gene (GST-II-27), which can be activated by exogenously applied safeners such as, for example, N,N-diallyl-2,2-dichloroacetamide (W0 93/01294) and which is operable in a large number of tissues of both monocots and dicots.

Particularly preferred are constitutive promoters, very particularly preferred seed-specific promoters, in particular the napin promoter and the USP promoter.

In addition, further promoters which make possible expression in further plant tissues or in other organisms such as, for example, E.coli bacteria, may be linked operably with the nucleic acid sequence to be expressed. Suitable plant promoters are, in principle, all of the above-described promoters.

The nucleic acid sequences present in the transgenic expression cassettes according to the invention or transgenic vectors can be linked operably with further genetic control sequences besides a promoter. The term genetic control sequences is to be understood in the broad sense and refers to all those sequences which have an effect on the establishment or the function of the expression cassette according to the invention. Genetic control sequences modify, for example, transcription and translation in prokaryotic or eukaryotic organisms. The transgenic expression cassettes according to the invention preferably encompass a plant-specific promoter 5'-upstream of the nucleic acid sequence to be expressed recombinantly in each case and, as additional genetic control sequence, a terminator sequence 3'-downstream, and, if appropriate, further customary regulatory elements, in each case linked operably with the nucleic acid sequence to be expressed recombinantly.

Genetic control sequences also encompass further promoters, promoter elements or minimal promoters capable of modifying the expression-controlling properties. Thus, genetic control sequences can, for example, bring about tissue-specific expression which is additionally dependent on certain stress factors. Such elements are, for example, described for water stress, abscisic acid (Lam E and Chua NH, J Biol Chem 1991; 266(26): 17131 -17135) and thermal stress (Schoffl F et al. (1989) Mol Gen Genetics 217(2-3):246-53).

Further advantageous control sequences are, for example, in the Gram-positive promoters amy and SPO2, and in the yeast or fungal promoters ADC1, MFa, AC, P-60, CYC1, GAPDH, TEF, rp28, ADH.

In principle all natural promoters with their regulatory sequences like those mentioned above may be used for the method according to the invention. In addition, synthetic promoters may also be used advantageously.

Genetic control sequences further also encompass the 5'-untranslated regions, introns or nonencoding 3'-region of genes, such as, for example, the actin-1 intron, or the Adh1-S intron 1, 2 and 6 (for general reference, see: The Maize Handbook, Chapter 116, Freeling and Walbot, Eds., Springer, New York (1994)). It has been demonstrated that these may play a significant role in regulating gene expression. Thus, it has been demonstrated that 5'-untranslated sequences can enhance the transient expression of heterologous genes. Translation enhancers which may be mentioned by way of example are the tobacco mosaic virus 5' leader sequence (Gallie et al. (1987) Nucl Acids Res 15:8693-8711) and the like. They may furthermore promote tissue specificity (Rouster J et al. (1998) Plant J 15:435-440).

The transient expression cassette can advantageously contain one or more of what are known as enhancer sequences in operable linkage with the promoter, and these make possible an increased recombinant expression of the nucleic acid sequence. Additional advantageous sequences such as further regulatory elements or terminators may also be inserted at the 3' end of the nucleic acid sequences to be expressed recombinantly. One or more copies of the nucleic acid sequences to be expressed recombinanly may be present in the gene construct.

Polyadenylation signals which are suitable as control sequences are plant polyadenylation signals, preferably those which correspond essentially to Agrobacterium tumefaciens T-DNA polyadenylation signals, in particular those of gene 3 of the T-DNA (octopine synthase) of the Ti plasmid pTiACHS (Gielen et al. (1984) EMBO J 3:835 et seq.) or functional equivalents thereof. Examples of particularly suitable terminator sequences are the OCS (octopin synthase) terminator and the NOS (nopaline synthase) terminator.

Control sequences are furthermore understood as those which make possible homologous recombination or insertion into the genome of a host organism, or removal from the genome. In the case of homologous recombination, for example, the coding sequence of the specific endogenous gene can be exchanged in a directed fashion for a sequence encoding a dsRNA. Methods such as the cre/lox technology permit the tissue-specific, possibly inducible, removal of the expression cassette from the genome of the host organism (Sauer B (1998) Methods. 14(4):381-92). Here, certain flanking sequences are added to the target gene (lox sequences), and these make possible removal by means of cre recombinase at a later point in time.

A recombinant expression cassette and the recombinant vectors derived from it may comprise further functional elements. The term functional element is to be understood in the broad sense and refers to all those elements which have an effect on generation, replication or function of the expression cassettes, vectors or transgenic organisms according to the invention. Examples which may be mentioned, but not by way of limitation, are:
a) Selection markers which confer resistance to a metabolism inhibitor such as 2-deoxyglucose-6-phosphate (WO 98/45456), antibiotics or biocides, preferably herbicides, such as, for example, kanamycin, G 418, bleomycin, hygromycin, or phosph nothricin and the like. Particularly preferred selection markers are those which confer resistance to herbicides. The following may be mentioned by way of example: DNA sequences which encode phosphinothricin acetyltransferases (PAT) and which inactivate glutamine synthase inhibitors (bar and pat gene), 5-enolpyruvylshikimate-3-phosphate synthase genes (EPSP synthase genes), which confer resistance to Glyphosate (N-(phosphonomethyl)glycine), the gox gene, which encodes Glyphosate-degrading enzyme (Glyphosate oxidoreductase), the deh gene (encoding a dehalogenase which inactivates dalapon), sulfonylurea- and imidazolinone-inactivating acetolactate synthases, and bxn genes which encode nitrilase enzymes which degrade bromoxynil, the aasa gene, which confers resistance to the antibiotic apectinomycin, the streptomycin phosphotransferase (SPT) gene, which permits resistance to streptomycin, the neomycin phosphotransferase (NPTII) gene, which confers resistance to kanamycin or geneticidin, the hygromycin phosphotransferase (HPT) gene, which confers resistance to hygromycin, the acetolactate synthase gene (ALS), which confers resistance to sulfonylurea herbicides (for example mutated ALS variants with, for example, the S4 and/or Hra mutation).
b) Reporter genes which encode readily quantifiable proteins and which allow the transformation efficacy or the expression site or time to be assessed via their color or enzyme activity. Very particularly preferred in this context are reporter proteins (Schenborn E, Groskreutz D. Mol Biotechnol. 1999; 13(1):29-44) such as the "green fluorescent protein" (GFP) (Sheen et al.(1995) Plant Journal 8(5):777-784), chloramphenicol transferase, a luciferase (Ow et al. (1986) Science 234:856-859), the aequorin gene (Prasher et al. (1985) Biochem Biophys Res Commun 126(3):1259-1268), β-galactosidase, with β-glucuronidase being very particularly preferred (Jefferson et al. (1987) EMBO J 6:3901-3907).
c) Replication origins which allow replication of the expression cassettes or vectors according to the invention in, for example, E.coli. Examples which may be mentioned are ORI (origin of DNA replication), the pBR322 ori or the P15A ori (Sambrook et al.: Molecular Cloning. A Laboratory Manual, 2nd ed. Cold Spring Harbor Laboratory Press, Cold Spring Harbor, NY, 1989).
d) Elements which are required for agrobacterium-mediated plant transformation such as, for example, the right or left border of the T-DNA, or the vir region.

To select cells which have successfully undergone homologous recombination or else cells which have succesfully been transformed, it is generally required additionally to introduce a selectable marker which confers resistance to a biocide (for example a herbicide), a metabolism inhibitor such as 2-deoxyglucose-6-phosphate (WO 98/45456) or an antibiotic to the cells which have successfully undergone recombination. The selection marker permits the selection of the transformed cells from untransformed cells (McCormick et al. (1986) Plant Cell Reports 5:81-84).

In addition, said recombinant expression cassette or vectors may comprise further nucleic acid sequences which do not encode a nucleic acid sequence SEQ ID NO: 1, SEQ ID NO: 3 or SEQ ID NO: 5 and whose recombinant expression leads to a further increase in fatty acid biosynthesis. By way of example, but not by limitation, such a proOIL nucleic acid sequence which is additionally expressed recombinantly can be selected from among nucleic acids encoding acetyl-CoA carboxylase (ACCase), glycerol-3-phosphate acyltransferase (GPAT), lysophosphatidate acyltransferase (LPAT), diacylglycerol acyltransferase (DAGAT) and phospholipid:diacylglycerol acyltransferase (PDAT). Such sequences are known to the skilled worker and are readily accessible from databases or suitable cDNA libraries of the respective plants.

An expression cassette according to the invention can advantageously be introduced into an organism or cells, tissues, organs, parts or seeds thereof (preferably into plants or plant cells, tissues, organs, parts or seeds) by using vectors in which the recombinant expression cassettes are present. The invention therefore furthermore relates to said recombinant vectors which encompass a recombinant expression cassette for a nucleic acid sequence SEQ ID NO: 1, SEQ ID NO: 3 or SEQ ID NO: 5.

For example, vectors may be plasmids, cosmids, phages, viruses or else agrobacteria. The expression cassette can be introduced into the vector (preferably a plasmid vector) via a suitable restriction cleavage site. The resulting vector is first introduced into E.coli. Correctly transformed E.coli are selected, grown, and the recombinant vector is obtained with methods known to the skilled worker. Restriction analysis and sequencing may be used for verifying the cloning step. Preferred vectors are those which make possible stable integration of the expression cassette into the host genome.

The invention furthermore relates to transgenic plant organisms or tissues, organs, parts, cells or propagation material thereof which comprise a SEQ ID NO: 3 or SEQ ID NO: 5 as defined above, a transgenic expression cassette for a SEQ ID NO: 3 or SEQ ID NO: 5 or a transgenic vector encompassing such an expression cassette.

Such a transgenic plant organism is generated, for example, by means of transformation or transfection of the corresponding proteins or nucleic acids. The generation of a transformed organism (or a transformed cell or tissue) requires introducing the DNA in question (for example the expression vector), RNA or protein into the host cell in question. A multiplicity of methods is available for this procedure, which is termed trans-formation (or transduction or transfection) (Keown et al. (1990) Methods in Enzymology 185:527-537). Thus, the DNA or RNA can be introduced for example directly by microinjection or by bombardment with DNA-coated microparticles. The cell may also be permeabilized chemically, for example with polyethylene glycol, so that the DNA may reach the cell by diffusion. The DNA can also be introduced by protoplast fusion with other DNA-comprising units such as minicells, cells, lysosomes or liposomes. Electroporation is a further suitable method for introducing DNA; here, the cells are permeabilized reversibly by an electrical pulse. Soaking plant parts in DNA solutions, and pollen or pollen tube transformation, are also possible. Such methods have been described (for example in Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73; Howell et al. (1980) Science 208:1265; Horsch et al.(1985) Science 227:1229-1231; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

In plants, the methods which have been described for transforming and regenerating plants from plant tissues or plant cells are exploited for transient or stable transformation. Suitable methods are, in particular, protoplast transformation by polyethylene glycol-induced DNA uptake, the biolistic method with the gene gun, what is known as the particle bombardment method, electroporation, the incubation of dry embryos in DNA-containing solution, and microinjection.

In addition to these "direct" transformation techniques, transformation may also be effected by bacterial infection by means of Agrobacterium tumefaciens or Agro bacterium rhizogenes and the transfer of corresponding recombinant Ti plasmids or Ri plasmids by infection with transgenic plant viruses. Agrobacterium-mediated transformation is best suited to cells of dicotyledonous plants. The methods are described, for example, in Horsch RB et al. (1985) Science 225: 1 229f).

When agrobacteria are used, the expression cassette is to be integrated into specific plasmids, either into a shuttle vector or into a binary vector. If a Ti or Ri plasmid is to be used for the transformation, at least the right border, but in most cases the right and left border, of the Ti or Ri plasmid T-DNA is linked to the expression cassette to be introduced as flanking region.

Binary vectors are preferably used. Binary vectors are capable of replication both in E.coli and in Agrobacterium. As a rule, they contain a selection marker gene and a linker or polylinker flanked by the right and left T-DNA border sequence. They can be transformed directly into Agrobacterium (Holsters et al. (1978) Mol Gen Genet 163:181-187). The selection marker gene, which is, for example, the nptII gene, which confers resistance to kanamycin, permits a selection of transformed agrobacteria. The Agrobacterium which acts as host organism in this case should already contain a plasmid with the vir region. The latter is required for transferring the T-DNA to the plant cells. An Agrobacterium transformed in this way can be used for transforming plant cells. The use of T-DNA for the transformation of plant cells has been studied intensively and described (EP 120 516; Hoekema, In: The Binary Plant Vector System, Offsetdrukkerij Kanters B.V., Alblasserdam, Chapter V; An et al. (1985) EMBO J 4:277-287). Various binary vectors, some of which are commercially available, such as, for example, pBI101.2 or pBIN19 (Clontech Laboratories, Inc. USA), are known.

Further promoters which are suitable for expression in plants have been described (Rogers et al. (1987) Meth in Enzymol 153:253-277; Schardl et al. (1987) Gene 61:1-11; Berger et al. (1989) Proc Natl Acad Sci USA 86:8402-8406).

Direct transformation techniques are suitable for any organism and cell type. In cases where DNA or RNA are injected or electroporated into plant cells, the plasmid used need not meet any particular requirements. Simple plasmids such as those from the pUC series may be used. If intact plants are to be regenerated from the transformed cells, it is necessary for an additional selectable marker gene to be present on the plasmid.

Stably transformed cells, i.e. those which contain the inserted DNA integrated into the DNA of the host cell, can be selected from untransformed cells when a selectable marker is part of the inserted DNA. By way of example, any gene which is capable of conferring resistance to antibiotics or herbicides (such as kanamycin, G 418, bleomycin, hygromycin or phosphinothricin and the like) is capable of acting as marker (see above). Transformed cells which express such a marker gene are capable of surviving in the presence of concentrations of such an antibiotic or herbicide which kill an untransformed wild type. Examples are mentioned above and preferably comprise the bar gene, which confers resistance to the herbicide phosphinothricin (Rathore KS et al. (1993) Plant Mol Biol 21(5):871-884), the nptII gene, which confers resistance to kanamycin, the hpt gene, which confers resistance to hygromycin, or the EPSP gene, which confers resistance to the herbicide Glyphosate. The selection marker permits selection of transformed cells from untransformed cells (McCormick et al. (1986) Plant Cell Reports 5:81-84). The plants obtained can be bred and hybridized in the customary manner. Two or more generations should be grown in order to ensure that the genomic integration is stable and hereditary.

The above-described methods are described, for example, in Jenes B et al.(1993) Techniques for Gene Transfer, in: Transgenic Plants, Vol. 1, Engineering and Utilization, edited by SD Kung and R Wu, Academic Press, pp.128-143, and in Potrykus (1991) Annu Rev Plant Physiol Plant Molec Biol 42:205-225). The construct to be expressed is preferably cloned into a vector which is suitable for transforming Agrobacterium tumefaciens, for example pBin19 (Bevan et al. (1984) Nucl Acids Res 12:8711f).

Once a transformed plant cell has been generated, an intact plant can be obtained using methods known to the skilled worker. For example, callus cultures are used as starting material. The development of shoot and root can be induced in this as yet undifferentiated cell biomass in the known fashion. The plantlets obtained can be planted out and used for breeding.

The skilled worker is familiar with such methods for regenerating plant parts and intact plants from plant cells. Methods which can be used for this purpose are, for example, those described by Fennell et al. (1992) Plant Cell Rep. 11: 567-570; Stoeger et al (1995) Plant Cell Rep. 14:273-278; Jahne et al. (1994) Theor Appl Genet 89:525-533.

"Transgenic", for example in the case of an OEP, refers to a nucleic acid sequence, an expression cassette or a vector comprising said OEP nucleic acid sequence or to an organism transformed with said nucleic acid sequence, expression cassette or vector or all those constructs established by recombinant methods in which either
a) the nucleic acid sequence encoding an OEP or
b) a genetic control sequence, for example a promoter which is functional in plant organisms, which is linked operably with said nucleic acid sequence under a)
are not in their natural genetic environment or have been modified by recombinant methods, it being possible for the modification to be, for example, a substitution, addition, deletion, inversion or insertion of one or more nucleotide residues. Natural genetic environment refers to the natural chromosomal locus in the source organism or the presence in a genomic library. In the case of a genomic library, the natural genetic environment of the nucleic acid sequence is preferably retained, at least to some extent. The environment flanks the nucleic acid sequence at least on one side and has a sequence length of at least 50 bp, preferably at least 500 bp, particularly preferably at least 1000 bp, very particularly preferably at least 5000 bp. A naturally occurring expression cassette, for example the naturally occurring combination of the promoter of a gene coding for an OEP with the corresponding OEP gene, becomes a transgenic expression cassette when the latter is modified by non-natural, synthetic ("artificial") methods such as, for example, a mutagenization. Such methods are described in US 5,565,350; WO 00/15815; see also above.

Host or starting organisms which are preferred as transgenic organisms are, above all, plants in accordance with the above definition. Included for the purposes of the invention are all genera and species of higher and lower plants of the Plant Kingdom, in particular plants which are used for obtaining oils, such as, for example, oilseed rape, sunflower, sesame, safflower, olive tree, soya, maize, wheat and nut species. Furthermore included are the mature plants, seed, shoots and seedlings, and parts, propagation material and cultures, for example cell cultures, derived therefrom. Mature plants refers to plants at any desired developmental stage beyond the seedling stage. Seedling refers to a young, immature plant at an early developmental stage.

The transgenic organisms can be generated with the above-described methods for the transformation or transfection of organisms.

The invention furthermore relates to the use of the transgenic organisms according to the invention and to the cells, cell cultures, parts - such as, for example, in the case of transgenic plant organisms roots, leaves and the like - and transgenic propagation material such as seeds or fruits which are derived therefrom for the production of foodstuffs or feedstuffs, pharmaceuticals or fine chemicals, in particular oils, fats, fatty acids or derivatives of these.

The invention now having been generally described will be more readily understood by reference to the following examples, which are included for the purpose of illustration only, and are not intended to limit scope of the present invention.

### Examples

### General methods:

Unless otherwise specified, all chemicals were from Fluka (Buchs), Merck (Darmstadt), Roth (Karlsruhe), Serva. (Heidelberg) and Sigma (Deisenhofen). Restriction enzymes, DNA-modifying enzymes and molecular biological kits were from Amersham-Pharmacia (Freiburg), Biometra (Göttingen), Roche (Mannheim), New England Biolabs (Schwalbach), Novagen (Madison, Wisconsin, USA), Perkin-Elmer (Weiterstadt), Qiagen (Hilden), Stratagen (Amsterdam, Netherlands), Invitrogen (Karlsruhe) and Ambion (Cambridgeshire, United Kingdom). The reagents used were employed in accordance with the manufacturer's instructions.

For example, oligonucleotides can be synthesized chemically in the known manner using the phosphoamidite method (Voet, Voet, 2nd edition, Wiley Press New York, pages 896-897). The cloning steps carried out for the purposes of the present invention such as, for example, restriction cleavages, agarose gel electrophoreses, purification of DNA fragments, transfer of nucleic acids to nitrocellulose and nylon membranes, linking DNA fragments, transformation of E. coli cells, bacterial cultures, multiplication of phages and sequence analysis of recombinant DNA, are carried out as decribed by Sambrook et al. (1989) Cold Spring Harbor Laboratory Press; ISBN 0-87969-309-6. Recombinant DNA molecules were sequenced using an ABI laser fluorescence DNA sequencer following the method of Sanger (Sanger et al. (1977) Proc Natl Acad Sci USA 74:5463-5467).

### Example 1

Decreased incorporation of [¹⁴C]Glycerol 3-Phosphate into lipids of a yeast strain in which the *YPR140w* gene was disrupted.

A haploid yeast (*Saccharomyces cerevisiae)* strain, (EUROSCARF accession no. Y15555; EUROSCARF, University Frankfurt, D-60439 Frankfurt, Germany,) with the *YPR140w* gene deleted, and the corresponding wild type (wt) strain By4742 (*MATα his3*Δ*1, leu2* Δ*0, lys2* Δ*0, ura3 Δ0*), were acquired from the EUROSCARF yeast strain collection. Yeast cells were grown in liquid YPD (1 % yeast extract, 2% peptone and 2% glucose). For preparing microsomal membranes two yeast strains, Y15555 and wt, were grown in 750 ml cultures in liquid YPD on rotary shaking at 30°C. The cultures were started by inoculation from overnight liquid-YPD pre-cultures to an OD₆₀₀ of 0.05 and the cells were harvested at an OD₆₀₀ of about 1.5, by centrifugation for five minutes at 3000rpm. The cell pellets were washed once in deionised water and frozen.

Cell pellets were thawed and washed with breaking buffer (0.6 M Mannitol, 20 mM Tris-HCl pH 7.6, 1 mM EDTA, 1 µg/ml Aprotinin, 0.7 µg/ml Pepstatin, 0.5 µg/ml Leupeptin) before being centrifuged for five minutes at 30000 rpm. The cells were then resuspended in breaking buffer (one ml of breaking buffer per g of wet cell pellet) and then broken by passing them 2-3 times through a French Press (Ammco SLM instruments, Urbane lllinios). The cells were diluted 1.5 times with breaking buffer and centrifuged at 1000g for five minutes at 4°C. The supernatant was then centrifuged two additional times, first at 5000g for 10 minutes then at 100,000 g for sixty minutes both at 4°C. The microsomal pellet was re-suspended in 20 mM Tris-HCl, pH 7.6 to a protein concentration of approximately 10mg/ml and homogenized with glass homogenizer. Microsomes were stored frozen at -70°C. Protein concentrations were determined using the BCA method (Pierce) with BSA as standard protein. The enzymatic incorporation of [¹⁴C]glycerol-3-phosphate were analysed by adding 50 µl of an reaction mixture (50 mM Tris-HCl pH 7.5, 8 mM NaF, 4 mM MgCl₂, 0.4 mM 10000 dpm/nmol [¹⁴C]-G3P, 4 mg/ml BSA, 0.2mM 16:0-CoA, 2 mM DTT) to 5µl of microsomal preparations in a final volume of 100 µl. After incubation at 30°C for 10 min the reactions were stopped by adding 400 µl of methanol/chloroform/ glacial acetic acid (50:50:1) and 100 µl of water followed by thoroughly shaking and centrifugation for twenty seconds at 13000 rpm. The bottom chloroform layer was removed and transferred into scintillation vials containing 4 ml of scintillation fluid (ethanol/ethanol 2:1, 0.4% 2(1-butlphenol)-5-(4-biph-enyl)-1, 3, 4-oxadizole). Radioactivity counting was carried out in a LKB Wallace 1290 Rackbeta Liquid scintillation counter.

The effect of deleting the *YPR140w* gene in yeast was studied on the *in vitro* biosynthesis of lipids by analysing the incorporation rate of [¹⁴C]glycerol-3phosphate into lipids. The glycerol-3-phospate acyitransferase (GPAT) reaction is the first step in the lipid biosynthesis in yeast, both for phospholipids and triacylglycerols. The lysophosphatidic acid formed by GPAT activity can be further metabolised into phosphatidic acid and diacylglycerol by other enzymes which are part of the so called Kennedy pathway which are also present in the microsomal membrane fraction. The method used in this experiment to analyse the incorporation of [¹⁴C]glycerol-3phosphate into lipids, is taking advantage of the fact that the labelled substrate is water soluble while the products are lipid soluble. This means that a simple chloroform extraction with scintillation counting of the chloroform phase will give the amount of incorporated [¹⁴C]glycerol-3phosphate into lipids, which is the same as the GPAT activity as all labelled lipid products formed has to pass the GPAT acylation step. The *YPR140w-*deletion and wild type yeast strains were grown in liquid cultures and microsomal membrane fractions were prepared from harvested cell pellets. Removing the gene had little or no effect on the growth rate as determined by optical density measurements. However, the incorporation of [¹⁴C]glycerol-3phosphate into lipids when microsomal membranes were incubated in the GPAT assay was clearly reduced to about 60% of wild type, see figure 1. These results indicate that the *YPR140w gene* might encode a GPAT enzyme or another type of protein that affects microsomal GPAT activity indirectly.

### Example 2

Increased incorporation of [¹⁴C]Glycerol 3-Phosphate into lipids in crude extracts of wt yeast over expressing the gene *YPR140w.*

For induced high-level expression of the *YPR140w* gene, a 1146 bp DNA fragment as described in SEQ ID NO: 1, from start to stop codon, was amplified from *S.cerevisiae* W303 genomic DNA by using a 1:1 mixture of *Taq* and *pfu* DNA polymerases with the 5' primer, ATGTCTTTTAGGGATGTCCTAGA, and the 3' primer, TCAATCATCCTTACCCTTTGGTT. The resulting PCR product was gel purified, incubated with Taq polymerase and TA-cloned into the pCR2.1-TOPO cloning vector (invitrogen). The *YPR140w* gene was then excised from the cloning vector by *Eco*RI digestion and cloned into the *Eco*RI behind the strong inducible GAL1 promotor in the multicopy plasmid pYES2 (Invitrogen), thus generating the plasmid pAN3. The wt yeast strain By4742 (*MATα his3Δ1, leu2 Δ0, lys2 Δ0, ura3 Δ0*), was transformed with pAN3 and pYES2 using standard protocol. Overnight precultures of the two yeast transformants, wt yeast containing pAN3 or pYES2, were grown in 15 ml of synthetic liquid media lacking uracil and supplemented with 2% Glucose (vol/vol). All yeast cultures were grown on rotary shaking at 30°C. The precultures were used to inoculate sixty ml of synthetic liquid media lacking uracil and supplemented with 3% glycerol and 3% lactate to a start OD₆₀₀ of 0.2. The sixty ml cultures were grown for forty-eight hours (OD₆₀₀ about 1.5) and then the *GAL1* over expression promotor was induced by the addition of galactose to a final concentration of 2% (vol/vol) and the cultures were shaken for a further 6 hours at 30°C. Cells were harvested (OD₆₀₀ about 2.5) by centrifugation five minutes at 3000 rpm. Pellets were washed once with water and frozen. Cell pellets were thawed, resuspended in ice-cold breaking buffer (20mM Tris-HCl pH 7.6, 1 mM EDTA, 1µg/ml Aprotinin, 0.7µg/ml Pepstatin, 0.5 µg/ml Leupeptin) to 1,5 ml and added to two ml screw cap tubes containing 1 ml of acid washed glass beads (diameter 0.45-0.5 mm). The tubes were shaken at 4°C for 3 minutes in a Minibeadbeater-8 (Techtum lab and then centrifuged for 10 minutes at 3000 rpm. The supernatant was harvested and stored at-70°C. Protein concentrations were determined using the BCA-method (Pierce) with BSA as protein standard. Enzymatic assays were run for ten minutes at 30°C. The samples (100µg of protein) were diluted to 50 µl with water and the assay was started by adding 50µl of assay mixture (50 mM Tris-HCl pH 7.5, 8 mM NaF, 4 mM MgCl₂, 0.4 mM 10000dpm/nmol [¹⁴C]-G-3-P, 4 mg/ml BSA, 0.2 mM 16:0-CoA, 2 mM DTT). The assays were stopped by adding 400 µl of methanol/chloroform/ glacial acetic acid (50:50:1) and 100 µl of water followed by thoroughly shaking and centrifugation for twenty seconds at 13000 rpm. The bottom chloroform layer was removed and transferred into scintillation vials containing 4 ml of scintillation fluid (ethanol/ethanol 2:1, 0.4% 2(1-butylphenol)-5-(4-biphenyl)-1,3, 4-oxa-dizole). Radioactivity counting was carried out in a LKB Wallace 1290 Rackbeta Liquid scintillation counter.

The effect of over expression of the YPR *140w* gene was studied on the rate of *in vitro* incorporation of [¹⁴C]glycerol-3phosphate into lipids by crude cellular extracts. Over expression of the YPR *140w* gene was achieved by cloning the gene behind the strong galactose inducible *GAL1* promotor and transforming the generated plasmid, pAN3, into wt yeast. The over expression plasmid, pYES2, without any gene behind the *GAL1* promoter was used as an "empty plasmid" control in the experiment. Incorporation of glycerol-3phosphate into lipids is catalyzed by the GPAT (glycerol 3-phosphate acyltransferase) enzyme, which utilizes acyl-CoA as acyldonor for the reaction and gives lysophosphatidic acid as product. The GPAT reaction is the first step in the lipid biosynthesis in yeast, both for phospholipids and triacylglycerols. The formed lysophosphatidic acid can be further metabolized into phosphatidic acid and diacylglycerol by other enzymes, which are also present in the crude cellular extracts and are part of the so-called Kennedy pathway. The method used in this experiment to analyze the GPAT activity, or the incorporation of [¹⁴C]glycerol-3phosphate into lipids, is taking advantage of the fact that the labeled substrate is water-soluble while the products are lipid soluble, and that all labeled lipid products formed have to pass the GPAT step. This means that a simple chloroform extraction with scintillation counting of the chloroform phase will give the amount of incorporated [¹⁴C]glycerol-3phosphate into lipids, which is the same as the GPAT activity.

Induced high-level over expression of the *YPR140* gene SEQ ID NO: 1 from the pAN3 plasmid in wt yeast increased the incorporation of [¹⁴C]glycerol-3phosphate into lipids 2.5 fold as compared to empty plasmid control, see figure 2. The increase in incorporation of [¹⁴C]glycerol-3phosphate into lipids is an effect of increased GPAT activity, but this does not mean that *the YPR140w* gene product necessarily has to be a GPAT. The in vitro assay is done in a crude extract containing a very complex mixture of enzymes and proteins. Over expression of *YPR140w* SEQ ID NO: 1 in wt yeast leads to an increased GPAT activity.

### Example 3

### Triacylglycerol accumulation in yeast cells expressing the YPR140w gene

For induced high-level expression of the *YPR140w* gene, a 1146 bp DNA fragment as described in SEQ ID NO: 1, from start to stop codons, was amplified from *S. cerevisiae* W303 genomic DNA by using a 1:1 mixture of *Taq* and *pfu* DNA polymerases with the 5' primer, ATGTCTTTTAGGGATGTCCTAGA, and the 3' primer, TCAATCATCCTTACCCTTTGGTT. The resulting PCR product was gel purified, incubated with Taq polymerase and TA-cloned into the pCR2.1-TOPO cloning vector (Invitrogen). The *YPR140w* gene was then excised from the cloning vector by *Eco*RI digestion and cloned into the *Eco*RI behind the strong inducible GAL1 promotor in the multicopy plasmid pYES2 (Invitrogen), thus generating the plasmid pAN3. The wild type yeast strain By4742 (*MATα his3α1, leu2 Δ0, lys2* Δ*0, ura3* Δ*0*), transformed with the pAN3 was cultivated at 30°C on a rotary shaker in synthetic medium (Sherman, F. et al., (1986) Laboratory Course Manual for Methods in Yeast Genetics, Cold Spring Harbor Lab. Press, Plainview, NY.) lacking uracil and supplemented with 2% (vol/vol) glycerol and 2% (vol/vol) ethanol. The *GAL1* promoter was induced after 24 hours of growth by the addition of 2% (wt/vol) final concentration of galactose. Cells were harvested after an additional 24 or 28 hours of growth. Wild type cells By4742, transformed with the empty vector (pYES2) and cultivated under identical conditions, were used as a control.

In order to quantify the total lipid content, 3 x 5 ml aliquots from yeast cultures were harvested by centrifugation, and the resulting pellets were washed with distilled water and lyophilised. The weight of the dried cells was determined, and the fatty acid content was quantified by conventional gas-liquid chromatography (GLC) analyses after conversion to methyl esters (Dahlqvist et al. (2000) Proc. Natl. Acad Sci. USA 97, 6487-6492).

The lipid content was then calculated as nmol fatty acids per mg dry weight. The lipid composition of the yeast was determined in cells harvested from 35-ml liquid cultures. The harvested yeast cells were re-suspended in 15 ml glass tubes in water to a final volume of 0.6 ml, to which 3.75 ml chloroform: methanol (1:2), 50 µl acetic acid, and 2 ml of glass beads (0.45-0.50 mm) were added. The yeast cells were disrupted by vigorous agitation (5 x 1 min) and the lipids were extracted into chloroform according to standard method (Bligh, E.G. and Dyer, W.J., Can. J. Biochem. Physiol. 37(1959), 911-917). The collected lipid fraction was divided in two parts and separated by TLC on Silica Gel 60 plates (Merck) in hexane / diethyl ether / acetic acid (70:30:1) for the quantification of neutral lipids, i.e. unesterified fatty acids (FA), diacylglycerols (DAG), triacylglycerols (TAG), and steryl esters (SE), and in chloroform / methanol / acetic acid: water (85: 15: 10: 3.5) for the quantification of the major polar lipids, i.e. phosphatidylinositol (PI), phosphatidylserine (PS), phosphatidylcholine (PC), and phosphatidylethanolamine (PE). The lipid areas were located by brief exposure to I₂ vapors and identified by means of appropriate standards. The different lipid classes were excised from the plates and fatty acid methyl esters were prepared by heating the excised material at 85°C for 60 min in 2% (vol/vol) sulfuric acid in dry methanol. The methyl esters were extracted and quantified by conventional gas-liquid chromatography (GLC) analyses as described in Dahlqvist et al., 2000.

The effect of high-level expression of the *YPR140w* gene SEQ ID NO: 1 on lipid accumulation was studied by transforming the wild-type yeast strain By4742 with the pAN3plasmid containing the *YPR140wgene,* under control of the galactose-induced *GAL1* promotor. The high-level expression of the *YPR140w* gene from this promoter did not retard the growth rate as determined by optical density measurements. The expression of the *YPR140w* gene was induced after 24 h and cells were harvested after an additional 24 hours (Exp.#1) or 28 hours (Exp.#2) of cultivation. The total lipid content was determined as nmol fatty acids per mg dry weight and was shown to be significantly higher in cells expressing the *YPR 140w* gene as compared to cells transformed with an empty vector. The total lipid content of the *YPR140w* (pAN3) transformants harvested 24 hours after induction (Exp.#1 in Fig. 3) was 16%, as compared to control cells (pYES2). Similarly the lipid content was 12% higher (Exp.#2 in Fig. 3) when harvested 28 hours after induction as compared to control cells. Furthermore, no major changes in the overall fatty acid composition occurred by the over expression of the *YPR140w* gene*.* The content of neutral lipid species was examined and this analysis clearly demonstrates that the major neutral lipid, triacylglycerol, is strongly elevated in cells over expressing the *YPR140w* (pAN3) as compared to control cells (table 3). The triacylglycerol content in the *YPR140w* (pAN3) transformant harvested 28 hours after induction (Exp#2 in table 3) was 62 %, as compared to the empty vector control. In cells harvested 24 hours after induction (Exp#1 in table 3) the triacylglycerol content was similarly elevated although less pronounced. It is therefore concluded that the triacylglycerol content is clearly increased in yeast cells by the over expression of the *YPR140wgene* SEQ ID NO: 1. The content of steryl esters is low in the control cells and was less affected by the over expression of this gene.

**Table 3.**

| | **Lipid content** | | | | | | | | | |
|---|---|---|---|---|---|---|---|---|---|---|
| | **(nmol FA per mg dry weight)** | | | | | | | | | |
| | **Polar lipids** | | **DAG** | | **FA** | | **TAG** | | **SE** | |
| | Exp#1 | Exp#2 | Exp#1 | Exp#2 | Exp#1 | Exp#2 | Exp#1 | Exp#2 | Exp#1 | Exp#2 |
| **pYES2** | 81 | 99 | 7.2 | 7.8 | 6.6 | 6.0 | 92 | 104 | 4.9 | 5.6 |
| **pAN3** | 72 | 67 | 9.7 | 5.0 | 6.3 | 1.3 | 130 | 169 | 5.9 | 7.0 |

The lipid content in yeast transformants. Yeast cells, were transformed with the pAN3 vector expressing the *YPR140w* SEQ ID NO: 1 from the GAL1 inducible promotor, and cells trans-formed with empty vector (pYES2) were used as control. The content of diacylglycerol (DAG), unesterified fatty acids (FA), triacylglycerol (TAG), and steryl esters (SE) was determined in yeast cells harvested 24 hours after induction and calculated as nmol fatty acid (FA) per mg dry cell weight.

A significant decrease in the overall polar lipid content was also observed due to the elevated expression of the *YPR140w* gene. The polar lipid content per dry cell weight decreased with up to 32% (table 4). Moreover, the relative distribution of the major polar lipid species as presented in table 1 differs between the overexpressor and the control cells. The relative content of phosphatidylinositol (PI) and phosphatidylethanolamine (PE) is decreased whereas phosphatidylcholine (PC) and phosphatidylserine (PS) is increased in the *YPR140w* transformant.

**Table 4.**

| | **Relative content of polar lipids** | | | |
|---|---|---|---|---|
| | **(%)** | | | |
| | **PI** | **PS** | **PC** | **PE** |
| pYES2 | 10,6 | 10,3 | 47,0 | 32,1 |
| pAN3 | 4,8 | 12,0 | 59,1 | 24,1 |

The relative composition (%) of phosphatidylinositol (PI), phosphatidylserine (PS), phosphatidylcholine (PC) and phosphatidylethanolamine (PE) in the different transformants harvested after 28 hours after induction. Yeast cells were transformed with the pAN3 vector expressing the *YPR140w* SEQ ID NO: 1 from the GAL1 inducible promoter. Yeast cells transformed with empty vector (pYES2) were used as control.

In summary, the triacylglycerol content in yeast cells expressing *YPR140w* SEQ ID NO: 1 from a strong promoter was increased with 61 % as compared to the control, which demonstrates the potential use of these genes in increasing the oil content in transgenic organisms.

### Example 4

### Identification of genes homologous to YPR140w in plants.

To identify possible homologues to the *YPR140w* yeast gene SEQ ID NO: 1, the NCBI database were searched for related plant sequences. From these BLAST searches two protein sequences in *Arabidopsis thaliana* were identified with 31,6 % (At140.1) and 26.3% (At140.2) sequence identity to the deduced amino acid sequence of the open reading frame *YPR140w* using the GAP programme. From an EST database the full-length sequences of the corresponding cDNAs were identified and named *At140.1* and *At140.2*. The Arabidopsis *At140.1* and *At140.2* genes were then PCR amplified from an Arabidopsis seedling cDNA library. The At140.1 gene was amplified from the cDNA by Pfu Turbo DNA polymerase (Stratagene, USA) with the use of the primers At140.1-S1: GTCGGTCTTTCTAACTGAATC and At140.1-A1:CCTGTGGGACTTAAACCTCA. The PCR product was diluted 10 times and used as a template in a second PCR with internal primers At140.1-S2:CAGAATGGGAATTCATTTTG and At140.1-A2: CTAACGGGAGTTTAACTTGCA. Similarly, the At140.2 gene was amplified from the cDNA by using the primers At140.2-S1: CTGGTCTCGTTTCTAATTG and At140.2-A1: CATGGCGAATCTAAACCGGAAC. The resulting DNA fragments were purified and cloned into pCR-Blunt II-TOPO (Invitrogen life technologies, USA), generating pCR-At140.1 and pCR-At140.2, respectively. The genes were verified by sequencing, see SEQ ID NO: 3 and SEQ ID NO: 5.

### Example 5

### Triacylglycerol accumulation in yeast cells expressing the plant genes At140.1 or At140.2

The *At140.1* and the *At140.2* genes SEQ ID NO: 3 and SEQ ID NO: 5 were excised from the cloning vectors pCR-At140.1 and pCR-At140.2 by BamHI and Notl digestions, respectively. The excised genes were then subcloned into the BamHl and Notl sites, respectively, behind the strong inducible GAL1 promotor in the multicopy plasmid pYES2 (Invitrogen, USA), thus generating the plasmids pY-At140.1 and pY-At140.2, respectively. The wild type yeast strain By4742 (*MATα his3Δ1, leu2 Δ0, lys2 Δ0, ura3 Δ0*), transformed with these plasmids were cultivated at 30°C on a rotary shaker in synthetic medium (Sherman et al., 1986) lacking uracil and supplemented with 2% (vol/vol) glycerol and 2% (vol/vol) ethanol. The *GAL1* promoter was induced after 24 hours of growth by the addition of 2% (wt/vol) final concentration of galactose. Cells were harvested after an additional 24 hours of growth. Wild type cells By4742 transformed with the empty vector, pYES2 and cultivated under identical conditions were used as a control. The lipid content of the yeast cells was determined as described in Example 3.

The effect of high-level expression of the *At140.1* (SEQ ID NO: 3) or the *At140.2* gene (SEQ ID NO: 5) on lipid accumulation was studied in yeast transformants expressing the *At140.1* or the *At140.2* gene, respectively, under control of the galactose-induced *GAL 1* promotor. The gene expression was induced after 24 hours and cells were harvested after an additional 24 hours of cultivation. High-level expression of these genes showed only minor effects on the growth rate as determined by optical density measurements. However, the total lipid content, determined as nmol fatty acids (FA) per mg yeast, was strongly elevated in the *Art140.1* or the *At140.2* transformants as compared to cells transformed with an empty vector. The total lipid content (Fig. 4) of the *At140.1* (pY-At140.1) and *At140.2* (pY-At140.2) transformants was 42% and 45% higher, respectively, as compared to control cells (pYES2). The increased lipid content is mainly explained by the strongly elevated triacylglycerol content in the *At140.1* and the *At140.2* transformants (table 5). The triacylglycerol content in these transformants was 79% and 92% higher, respectively, as compared to the empty vector control. The polar lipid content was slightly lowered and contents of unesterified fatty acids and diacylglycerols were increased whereas the steryl ester content was not affected by the over expression of the Arabidopsis genes, *At140.1* and *At140.2.*

**Table 5.**

| | **Lipid content** | | | | |
|---|---|---|---|---|---|
| | **(nmol FA per mg dry weight)** | | | | |
| | **Polar lipids** | **DAG** | **FA** | **TAG** | **SE** |
| **pYES2** | 83 | 5.0 | 3.6 | 73 | 6.7 |
| **PY-At140.1** | 75 | 9.1 | 21 | 131 | 7.3 |
| **PY-At140.2** | 70 | 8.8 | 24 | 140 | 6.8 |

The lipid content in yeast transformants. Yeast cells, were transformed with the pY-At140.1 or the pY-140.2 vector expressing the *At140.1* or the *At140.2* from the GAL1 inducible promoter. Yeast cells transformed with empty vector (pYES2) were used as control. The content of diacylglycerol (DAG), unesterified fatty acids (FA), triacylglycerol (TAG), and steryl esters (SE) was determined in yeast cells harvested 24 hours after induction and calculated as nmol fatty acid (FA) per mg dry cell weight.

The major polar lipids in yeast are phosphatidylinositol (PI), phosphatidylserine (PS), phosphatidylcholine (PC) and phosphatidylethanolamine (PE). The effects on the relative distribution of these major polar lipid classes by the over expression of the *At140.1* or the *At140.2* gene are presented in table 6. The most significant effect was observed in the phosphatidylinositol (PI) content of the *At140.1* or the *At140.2* transformants, with a 39% and a 33% decrease in the relative content as compared to control cells.

**Table 6.**

| | **Relative content of polar lipids** | | | |
|---|---|---|---|---|
| | **(%)** | | | |
| | **PI** | **PS** | **PC** | **PE** |
| **pYES2** | 11.0 | 12.2 | 54.3 | 29.7 |
| **pY-At140.1** | 6.7 | 11.1 | 48.7 | 33.4 |
| **pY-At140.2** | 7.4 | 11.9 | 47.5 | 33.2 |

The relative composition (%) of phosphatidylinositol (PI), phosphatidylserine (PS), phosphatidylcholine (PC) and phosphatidylethanolamine (PE) in the different transformants harvested after 24 hours after induction. Yeast cells were transformed with the pY-At140.1 or the pY-At140.2 vector expressing the *At140.1* or the *At140.2* gene, respectively, from the GAL1 inducible promoter. Yeast cells transformed with empty vector (pYES2) were used as control.

The effect on the fatty acid profile by the over expression of the *At140.1* or the *At140.2* gene were also analysed. Only minor changes in response to the over expression of these genes on the fatty acid composition of the polar lipids were observed (data not shown). However, the levels of oleic (18:1) acid increased and the palmitic (16:0) decreased in triacylglycerols accumulated in yeast cells expressing the *At140.1* or the *At140.2* gene (table 7). A minor increase in the stearic acid (18:0) content was also observed.

**Table 7.**

| | **Relative fatty acid composition in triacylglycerol** | | | |
|---|---|---|---|---|
| | **(%)** | | | |
| | **16:0** | **16:1** | **18:0** | **18:1** |
| **pYES2** | 30.5 | 31.5 | 13.2 | 24.8 |
| **pY-At140.1** | 14.5 | 31.3 | 15.4 | 38,3 |
| **pY-At140.2** | 14.2 | 31.4 | 15.0 | 39.3 |

The fatty acid composition of triacylglycerols in yeast cells transformed with the pY-At140.1 or the pY-At140.2 vector expressing the *At140.1* or the *At140.2* gene, respectively, from the GAL1 inducible promotor. Yeast cells transformed with empty vector (pYES2) were used as control. Major fatty acids present in yeast are palmitic (16:0), palmitoleic (16:1), stearic (18:0), and oleic (18:1) acid.

In summary, the triacylglycerol content in yeast cells expressing the *At140.1* (SEQ ID NO: 3) or the *At140.2* gene (SEQ ID NO: 5) from a strong promoter was increased with 78 or 92% as compared to the control, which demonstrates the potential use of these genes in increasing the oil content in transgenic organisms.

### Example 6

### Transgenic plants expressing the At140.1, At140.2 or YPR140w gene

*Cloning and analysis of oil content in plant:* For induced high-level expression of the *At140.1* (SEQ ID NO: 3) *or At140.2* gene (SEQ ID NO: 5) in plants, the genes were cloned into a binary vector system under the control of either a seed-specific or a constitutive promoter. An *Agrobacterium*-mediated plant transformation binary vector system was applied, including the primary cloning vector pART7-35S with a CaMV35S promoter or pART7-Nap with the seed specific napin promoter from *Brassica napus* (Josefsson, L. G. et al., J. Biol. Chem. 262 (1987), 12196-12201) coupled with a transcriptional terminator sequence and a binary Ti vector pGII0229 (Hellens, R.P. et al., Plant Mol. Biol. 42 (2000), 819-832). A DNA fragment, containing gene At140.1, was excised by BamHl and EcoRV from the cloning vector pCR-At140.1 (described in example 4) and was then subcloned into BamHl and Smal treated pART7-35S or pART7-Nap, generating p35S-At140.1 and pNap-At140.1, respectively. From these plasmids the expression cassettes containing the promoter, the At140.1 gene and the transcriptional terminator sequence was excised by Notl cleavage. The fragments were then inserted into the Notl site of the pGl10229 plasmid, generating the vectors pGII-35S-At140.1 and pGII-Nap-At140.1, respectively.

Plant expression vectors with the gene At140.2 expressed from a constitutive CaMV35S or from a seed specific napin promoter were constructed. First the Notl site was first deleted from the pCR-At140.2 by cutting with Not1, filling and re-ligation. Thereafter the *At140.2* gene was released by Hindlll and Xbal and inserted into the corresponding sites of the plasmid pART7-35S or pART7-Nap, generating plasmids p35S-At140.2 and pNap-At140.2, respectively. The expression cassettes were then excised from these plasmids and introduced into NotI site of pGII0229, generating plasmids pGll-35S-At140.2 and pGll-Nap-At140.2, respectively.

A plant expression vector with the gene *YPR140w* (SEQ ID No.1) was constructed by excision of the nucleotide stretch carrying *YPR140w* with EcoRI from the plasmid pAN3 (see example 3). The ends of the polynucleotide were filled-in using Klenow fragment creating blunt ends. Using the polynucleotide prepared in this way and the plant expression vector pSUN300-USP, which was cut with Stul and dephosporylated, the *YPR140wgene* was introduced into a plant expression vector under the control of the seed specific USP promoter by blunt end ligation of the polynucleotide and the opened pSun300-USP vector.

The plant expression vectors were introduced into *Agrobacterium tumefaciens* GV3101 using standard methods. Using floral dip essentially as described by Clough and Bent, 1998, plants were transformed with *Agrobacterium tumefaciens* GV3101 harboring the plasmid pGll-35S-At140.1, pGll-Nap-At140.1, pGll-35S-At140.2 and pGll-Nap-At140.2 respectively. Entire plants (inflorescence and rosette) were submerged for 20-30 sec in the infiltration media consisting of 5% sucrose, 0.02% Silwet L-77 (Osi Specialties, Danbury, CT) and re-suspended transformed *A. tumefasciens* cells. Plants were then transferred to a growth chamber with a photoperiod of 16 h of light at 21°C and 8 h of dark at 18°C (70% humidity). The T1 seeds were collected from mature plants. Subsequently transformed plants were identified on selection media by growing T1 seeds on MS-agar plates supplemented with BASTA (gulfosinate ammonium) 15 mg/l, cefotaxime 50 mg/L. Alternatively T1 plants were grown on soil trays and sprayed three times at an interval of two days with 0.4% BASTA.

Plant transformation with the vector pSUN300-USP-YPR140w was performed as described for the vectors carrying the At140.1 and At140.2 genes. For the transformants created using pSun300-USP-YPR140w transgenic plants were identified by germination on selection media containing kanamycin, since the pSun300 vector harbours the nptll gene as selection marker.

Plant lipids can be extracted from plant material as described by Cahoon et al. (1999, Proc. Natl. Acad. Sci. USA 96, 22:12935-12940) and Browse et al. (1986, Anal. Biochemistry 442:141-145). Qualitative and quantitative lipid or fatty acid analysis is described in Christie, William W., Advances in Lipid Methodology. Ayr/Scotland :Oily Press. - (Oily Press Lipid Library; Christie, William W., Gas Chromatography and Lipids. A Practical Guide - Ayr, Scotland :Oily Press, 1989 Repr. 1992. - IX,307).

T2 seeds from the *Arabidopsis* plants expressing the *YPR140w* gene from yeast were analysed for their seed oil content by the use of conventional gas-liquid chromatography (GLC) and calculated as mg fatty acids per mg dry seeds. Fig. 5 shows the seed lipid content of *Arabidopsis thaliana* T2 seeds expressing the gene YPR140w under the control of the seed-specific USP promoter (white bars). Error bars mark the deviation between two independent extractions The striped bar represents the average value of the four empty -vector control plants grown simultaneously, its error bar indicates the deviation of the average seed oil content of the control plants. Seed lipid content is calculated as mg fatty acids per g seeds (dry weight).

The expression of YPR140w in Arabidopsis plants led to an increase of seed oil content.

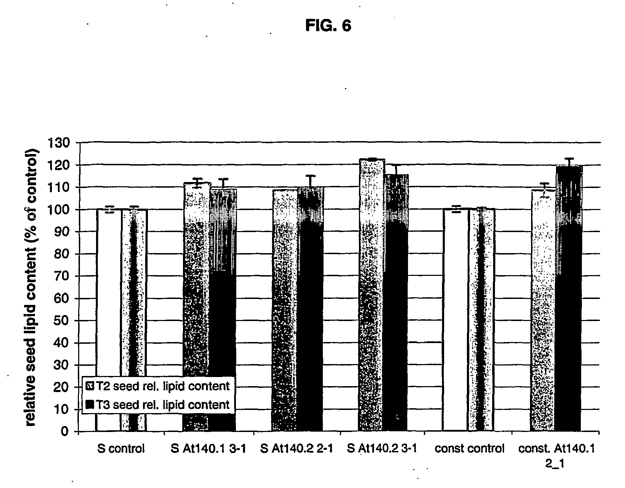

For the plants overexpressing the AT140.1 and At140.2 genes the oil content of T2 and T3 seeds of the *Arabidopsis* transformants was analyzed by the use of conventional gas-liquid Chromatography (GLC), calculated as mg fatty acids per mg dry seeds and standardized to the values for the simultaneously grown controls. Fig. 6 shows the seed oil content of *Arabidopsis thaliana* T2 (grey bars) and T3 seeds (black bars) of plants expressing At140.1 and Art140.2 under the control of a seed specific promoter ('S', left half of the graph) or a constitutive promoter ('const'; right columns). White bars represent the T2 controls, bars with a grey shaded centre mark the corresponding T3 controls. Error bars represent the standard deviation of 2 independent extractions for T2 seeds. For T3 seeds the error bars shown indicate the standard error of the mean of the average seed lipid values of at least 11 plants per line, analysed in 2 independent measurements. All values are shown as percentage of the average of the corresponding control plants. As control, seeds from wild type plants and plants carrying an empty vector were used. The over expression of the At140.1 (SEQ ID NO: 3) or the At140.2 gene (SEQ ID NO: 5) in Arabidopsis plants resulted in an increase in total seed lipid content in both generations.

## Claims

1. A method of increasing the total oil content in a plant organism or a tissue, organ, part, cell or propagation material thereof, comprising
a) the transgenic expression of a polypeptide SEQ ID NO: 2, SEQ ID NO: 4 or SEQ ID NO : 6 in said plant organism or in a tissue, organ, part, cell or propagation material thereof, and
b) the selection of plant organisms in which - in contrast to or comparison with the starting organism - the total oil content in said plant organism or in a tissue, organ, part, cell or propagation material thereof is increased.

2. The method as claimed in claim 1, wherein the oil biosynthesis enhancing protein is encoded by a nucleic acid sequence selected from the group consisting of:
a) a nucleic acid sequence comprising a nucleotide sequence which is at least 60% identical to the nucleic acid sequence of SEQ ID NO : 1, SEQ ID NO : 3 or SEQ ID NO: 5;
b) a nucleic acid sequence comprising a fragment of at least 30 nucleotides of a nucleic acid sequence comprising the nucleotide sequence of SEQ ID NO:1. SEQ ID NO: 3 or SEQ ID NO : 5;
c) a nucleic acid sequence which encodes a polypeptide comprising an amino acid sequence at least about 60% identical to the amino acid sequence of SEQ ID NO : 2, SEQ ID NO : 4 or SEQ ID NO 6 and
d) a nucleic acid sequence which encodes a fragment of a polypeptide comprising the amino acid sequence of SEQ ID NO : 2, SEQ ID NO : 4 or SEQ ID NO : 6 or wherein the fragment comprises at least 10 contiguous amino acid residues of the amino acid sequence of SEQ ID NO : 2, SEQ ID NO : 4 or SEQ ID NO : 6,

3. A method as claimed in claim 1 or 2, wherein the plant is an oil crop.

4. A method as claimed in claim 1 or 2, wherein the total oil content in the seed of a plant is increased.

5. An expression cassette comprising in combination with a regulatory sequence a nucleic acid sequence selected from the group consisting of:
a) a nucleic acid sequence comprising a nucleotide sequence which is at least 60% identical to the nucleotide sequence of SEQ ID NO : 3 or SEQ ID NO : 5,
b) a nucleic acid sequence comprising a fragment of at least 30 nucleotides of a nucleic acid sequence comprising the nucleotide sequence of SEQ ID NO : 3 or SEQ ID NO:5,
c) a nucleic acid sequence which encodes a polypeptide comprising an amino acid sequence at least about 60% identical to the amino acid sequence of SEQ ID NO : 4 or SEQ ID NO : 6, or
d) a nucleic acid sequence which encodes a fragment of a polypeptide comprising the amino acid sequence of SEQ ID NO : 4 or SEQ ID NO : 6 wherein the fragment comprises at least 10 contiguous amino acid residues of the amino acid sequence of SEQ ID NO: 4 or SEQ ID NO : 6
wherein said regulatory sequence is capable of mediating expression of said nucleic acid sequence in a plant.

6. An expression cassette according to claim 5, wherein said nucleic acid sequence encodes a polypeptide comprising the amino acid sequence set forth in SEQ ID NO : 4 or SEQ ID NO : 6.

7. An expression cassette as claimed in claim 5 or 6, wherein the promoter is a seed-specific promotor.

8. A genetically modified plant organism or tissue, organ, part, cell or propagation material thereof, comprising a polypeptide as defined in SEQ ID NO 4 or SEQ ID NO 6 or an expression cassette as claimed in any of claims 5 to 7.

9. A genetically modified plant organism as claimed in claim 8, wherein the plant organism is selected from the group of the oil crops consisting of Borvago officinalis, Brassica campestris, Brassica napus, Brassica rapa, Cannabis sativa, Carthamus tinctorius, Cocos nucifera, Crambe abyssinica, Cuphea species, Elaeis guinensis, Elaeis oleifera, Glycine max, Gossypium hirsutum, Gossypium barbadense, Gossypium herbaceum, Helianthus annuus, Linum usitatissimum, Oenothera biennis, Olea europaea, Oryza sativa, Ricinus communis, Sesamum Indicum, Triticum species, Zea mays, walnut and almond.

10. The use of a genetically modified plant organism or tissue, organ, part, cell or propagation material thereof as claimed in claim 8 or 9 for the production of oils, fats, free fatty acids or derivatives of the above.

## Patentansprüche

1. Verfahren zur Erhöhung des Gesamtölgehalts in einem pflanzlichen Organismus oder einem seiner Gewebe, Organe, Teile, Zellen oder Vermehrungsmaterialien, umfassend die folgenden Schritte:
a) transgene Expression eines Polypeptids SEQ ID NO : 2, SEQ ID NO : 4 oder SEQ ID NO : 6 in diesem pflanzlichen Organismus oder einem seiner Gewebe, Organe, Teile, Zellen oder Vermehrungsmaterialien, sowie
b) Selektion von pflanzlichen Organismen, in denen - im Gegensatz bzw. im Vergleich zu dem Ausgangsorganismus - der Gesamtölgehalt in diesem pflanzlichen Organismus oder in einem seiner Gewebe, Organe, Teile, Zellen oder Vermehrungsmaterialien erhöht ist.

2. Verfahren nach Anspruch 1, wobei das Protein, das die Ölbiosynthese fördert, von einer Nukleinsäuresequenz codiert wird, die ausgewählt ist aus der Gruppe:
a) Nukleinsäuresequenz umfassend eine Nukleotidsequenz, mit mindestens 60% Identität zu der Nukleinsäuresequenz gemäß SEQ ID NO : 1, SEQ ID NO : 3 oder SEQ ID NO : 5;
b) Nukleinsäuresequenz umfassend ein Fragment mit mindestens 30 Nukleotiden einer Nukleinsäuresequenz umfassend die Nukleotidsequenz gemäß SEQ ID NO : 1, SEQ ID NO : 3 oder SEQ ID NO : 5;
c) Nukleinsäuresequenz, die für ein Polypeptid codiert, das eine Aminosäuresequenz mit mindestens ungefähr 60% Identität zu der Aminosäuresequenz gemäß SEQ ID NO : 2, SEQ ID NO : 4 oder SEQ ID NO : 6 umfaßt, und
d) Nukleinsäuresequenz, die für ein Fragment eines Polypeptids umfassend die Aminosäuresequenz gemäß SEQ ID NO : 2, SEQ ID NO : 4 oder SEQ ID NO : 6 codiert oder wobei das Fragment mindestens 10 aufeinanderfolgende Aminosäurereste der Aminosäuresequenz gemäß SEQ ID NO : 2, SEQ ID NO : 4 oder SEQ ID NO : 6 umfaßt.

3. Verfahren nach Anspruch 1 oder 2, wobei es sich bei der Pflanze um eine Ölfrucht handelt.

4. Verfahren nach Anspruch 1 oder 2, wobei der Gesamtölgehalt im Samen einer Pflanze erhöht ist.

5. Expressionskassette umfassend eine Nukleinsäuresequenz aus der Gruppe bestehend aus:
a) Nukleinsäuresequenz umfassend eine Nukleotidsequenz, mit mindestens 60% Identität zu der Nukleotidsequenz gemäß SEQ ID NO : 3 oder SEQ ID NO : 5;
b) Nukleinsäuresequenz umfassend ein Fragment mit mindestens 30 Nukleotiden einer Nukleinsäuresequenz umfassend die Nukleotidsequenz gemäß SEQ ID NO : 3 oder SEQ ID NO : 5;
c) Nukleinsäuresequenz, die für ein Polypeptid codiert, das eine Aminosäuresequenz mit mindestens ungefähr 60% Identität zu der Aminosäuresequenz gemäß SEQ ID NO : 4 oder SEQ ID NO : 6 umfaßt, und
d) Nukleinsäuresequenz, die für ein Fragment eines Polypeptids umfassend die Aminosäuresequenz gemäß SEQ ID NO : 4 oder SEQ ID NO : 6 codiert, wobei das Fragment mindestens 10 aufeinanderfolgende Aminosäurereste der Aminosäuresequenz gemäß SEQ ID NO : 4 oder SEQ ID NO : 6 umfaßt,
in Kombination mit einer Regulationssequenz, wobei die Regulationssequenz fähig ist, die Expression der Nukleinsäuresequenz in einer Pflanze zu vermitteln.

6. Expressionskassette nach Anspruch 5, wobei die Nukleinsäuresequenz für ein Polypeptid umfassend die Aminosäuresequenz gemäß SEQ ID NO : 4 oder SEQ ID NO : 6 codiert.

7. Expressionskassette nach Anspruch 5 oder 6, wobei es sich bei dem Promoter um einen samenspezifischen Promoter handelt.

8. Genetisch modifizierter pflanzlicher Organismus oder eines seiner Gewebe, Organe, Teile, Zellen oder Vermehrungsmaterialien, umfassend ein Polypeptid nach SEQ ID NO : 4 oder SEQ ID NO : 6 oder eine Expressionskassette nach einem der Ansprüche 5 bis 7.

9. Genetisch modifizierter pflanzlicher Organismus nach Anspruch 8, wobei der pflanzliche Organismus aus der Gruppe der Ölfrüchte bestehend aus Borago officinalis, Brassica campestris, Brassica napus, Brassica rapa, Cannabis sativa, Carthamus tinctorius, Cocos nucifera, Crambe abyssinica, Cuphea species, Elaeis guinensis, Elaeis oleifera, Glycine max, Gossypium hirsutum, Gossypium barbadense, Gossypium herbaceum, Helianthus annuus, Linum usitatissimum, Oenothera biennis, Olea europaea, Oryza sativa, Ricinus communis, Sesamum indicum, Triticum species, Zea mays, Walnuß und Mandel stammt.

10. Verwendung eines genetisch modifizierten pflanzlichen Organismus oder eines seiner Gewebe, Organe, Teile, Zellen oder Vermehrungsmaterialien nach Anspruch 8 oder 9 zur Herstellung von Ölen, Fetten, freien Fettsäuren oder Derivaten der genannten Substanzen.

## Revendications

1. Procédé permettant d'augmenter la teneur en huile totale dans un organisme végétal, ou dans un tissu, un organe, une partie, une cellule, ou une matière de propagation associée, ce procédé comprenant :
a) l'expression transgénique d'un polypeptide SEQ ID n° 2, SEQ ID n° 4 OU SEQ ID n° 6 dans ledit organisme végétal ou dans un tissu, un organe, une partie, une cellule, ou une matière de propagation associée, et
b) la sélection d'organismes végétaux dans lesquels - par contraste ou par comparaison avec l'organisme de départ - la teneur en huile totale dans ledit organisme végétal ou dans un tissu, un organe, une partie, une cellule ou une matière de propagation associée est augmentée.

2. Procédé selon la revendication 1, dans lequel la protéine d'amélioration de la biosynthèse d'huile est codée par une séquence d'acide nucléique sélectionnée parmi le groupe se composant de :
a) une séquence d'acide nucléique comprenant une séquence nucléotidique qui est au moins identique à 60 % à la séquence d'acide nucléique de SEQ ID n° 1, SEQ ID n° 3 ou SEQ ID n° 5 ;
b) une séquence d'acide nucléique comprenant un fragment d'au moins 30 nucléotides d'une séquence d'acide nucléique comprenant la séquence nucléotidique de SEQ ID n° 1, SEQ ID n° 3 ou SEQ ID n° 5 ;
c) une séquence d'acide nucléique qui code un polypeptide comprenant une séquence d'acide aminé qui est au moins identique à 60 % environ à la séquence d'acide aminé de SEQ ID n° 2, SEQ ID n° 4 ou SEQ ID n° 6 ; et
d) une séquence d'acide nucléique qui code un fragment d'un polypeptide comprenant la séquence d'acide aminé de SEQ ID n° 2, SEQ ID n° 4 ou SEQ ID n° 6, où le fragment comprend au moins 10 résidus d'acide aminé contigus de la séquence d'acide aminé de SEQ ID n° 2, SEQ ID n° 4 ou SEQ ID n° 6.

3. Procédé selon la revendication 1 ou 2, dans lequel la plante est une plante oléagineuse.

4. Procédé selon la revendication 1 ou 2, dans lequel la teneur en huile totale dans la graine d'une plante est augmentée.

5. Cassette d'expression comprenant, en combinaison avec une séquence de régulation, une séquence d'acide nucléique sélectionnée parmi le groupe se composant de :
a) une séquence d'acide nucléique comprenant une séquence nucléotidique qui est au moins identique à 60 % à la séquence nucléotidique de SEQ ID n° 3 ou SEQ ID n° 5,
b) une séquence d'acide nucléique comprenant un fragment d'au moins 30 nucléotides d'une séquence d'acide nucléique comprenant la séquence nucléotidique de SEQ ID n° 3 ou SEQ ID n° 5,
c) une séquence d'acide nucléique qui code un polypeptide comprenant une séquence d'acide aminé qui est au moins identique à 60 % environ à la séquence d'acide aminé de SEQ ID n° 4 ou SEQ ID n° 6, ou
d) une séquence d'acide nucléique qui code un fragment d'un polypeptide comprenant la séquence d'acide aminé de SEQ ID n° 4 ou SEQ ID n° 6, où le fragment comprend au moins 10 résidus d'acide aminé contigus de la séquence d'acide aminé de SEQ ID n° 4 ou SEQ ID n° 6.
où ladite séquence de régulation peut induire l'expression de ladite séquence d'acide nucléique dans une plante.

6. Cassette d'expression selon la revendication 5, dans laquelle ladite séquence d'acide nucléique code un polypeptide comprenant la séquence d'acide aminé présentée dans SEQ ID n° 4 ou SEQ ID n° 6.

7. Cassette d'expression selon la revendication 5 ou 6, dans laquelle le promoteur est un promoteur spécifique des graines.

8. Organisme ou tissu, organe, partie, cellule ou matière de propagation associée, cet organisme étant un organisme végétal génétiquement modifié, comprenant un polypeptide tel que défini dans SEQ ID n° 4 ou SEQ ID n° 6 ou dans une cassette d'expression selon une des revendications 5 à 7.

9. Organisme végétal génétiquement modifié selon la revendication 8, dans lequel l'organisme végétal est sélectionné parmi le groupe des plantes oléagineuses se composant de Borago officinalis, Brassica campestris, Brassica napus, Brassica rapa, Cannabis sativa, Carthamus tinctorius, Cocos nucifera, Crambe abyssinica, Cuphea species, Elæis guinensis, Elaeis oleifera, Glycine max, Gossypium hirsutum, Gossypium barbadense, Gossypium herbaceum, Helianthus annuus, Linum usitatissimum, Oenothera biennis, Olea europæa, Oryza sativa, Ricinus communis, Sesamum indicum, Triticum species, Zea mays, noix et amande.

10. Utilisation d'un organisme végétal - ou tissu, organe, partie, cellule ou matière de propagation associée - génétiquement modifié selon la revendication 8 ou 9, destiné à la production d'huiles, de graisses, d'acides gras libres ou des dérivés de ceux-ci.


### SEQUENCE LISTING

<110> BASF Plant Science GmbH
<120> Use of genes for increasing the oil content in plants
<130> PF54384
<140> AE20020908
   <141> 2003-04-14
<160> 35
<170> PatentIn Ver. 2.1
<210> 1
   <211> 1146
   <212> DNA
   <213> Saccharomyces cerevisiae
<220>
   <221> CDS
   <222> (1)..(1146)
<400> 1
   atg tct ttt agg gat gtc cta gaa aga gga gat gaa ttt tta gaa gcc    48
   Met Ser Phe Arg Asp Val Leu Glu Arg Gly Asp Glu Phe Leu Glu Ala
    1               5                  10                  15
   tat ccc aga aga agc ccc ctt tgg aga ttt ctt tca tac agt aca tca    96
   Tyr Pro Arg Arg Ser Pro Leu Trp Arg Phe Leu Ser Tyr Ser Thr Ser
               20                  25                  30
   tta ctg acc ttc ggt gta tca aaa ctg ctt ctt ttc aca tgc tat aat   144
   Leu Leu Thr Phe Gly Val Ser Lys Leu Leu Leu Phe Thr Cys Tyr Asn
           35                  40                  45
   gtc aaa ttg aat ggt ttt gaa aaa tta gaa act gcc ttg gaa cgt tcc   192
   Val Lys Leu Asn Gly Phe Glu Lys Leu Glu Thr Ala Leu Glu Arg Ser
       50                  55                  60
   aaa agg gaa aat aga ggc ctt atg acg gtc atg aac cat atg agt atg   240
   Lys Arg Glu Asn Arg Gly Leu Met Thr Val Met Asn His Met Ser Met
   65                  70                  75                  80
   gtc gat gat ccg tta gtt tgg gca aca cta cca tat aag tta ttt acg   288
   Val Asp Asp Pro Leu Val Trp Ala Thr Leu Pro Tyr Lys Leu Phe Thr
                   85                  90                  95
   tct ttg gac aac ata aga tgg tct ttg ggt gca cat aat att tgc ttt   336
   Ser Leu Asp Asn Ile Arg Trp Ser Leu Gly Ala His Asn Ile Cys Phe
               100                 105                 110
   caa aat aaa ttt ctg gcc aac ttt ttc tca ctt ggc caa gtc ctt tca   384
   Gln Asn Lys Phe Leu Ala Asn Phe Phe Ser Leu Gly Gln Val Leu Ser
           115                 120                 125
   aca gaa aga ttt ggg gtg ggc cca ttt caa ggt tct ata gat gct tca   432
   Thr Glu Arg Phe Gly Val Gly Pro Phe Gln Gly Ser Ile Asp Ala Ser
       130                 135                 140
   ata aga ttg tta agc cct gac gac act tta gac ttg gaa tgg acc cct    480
   Ile Arg Leu Leu Ser Pro Asp Asp Thr Leu Asp Leu Glu Trp Thr Pro
   145                 150                 155                 160
   cac tct gag gtc tct tct tcg cta aaa aaa gcc tac tcc ccg ccc ata    528
   His Ser Glu Val Ser Ser Ser Leu Lys Lys Ala Tyr Ser Pro Pro Ile
                   165                 170                 175
   ata agg tcg aag cca tct tgg gtc cat gtt tat cca gaa gga ttt gta    576
   Ile Arg Ser Lys Pro Ser Trp Val His Val Tyr Pro Glu Gly Phe Val
               180                 185                 190
   cta caa tta tat ccg cct ttt gaa aat tcg atg agg tat ttt aaa tgg    624
   Leu Gln Leu Tyr Pro Pro Phe Glu Asn Ser Met Arg Tyr Phe Lys Trp
           195                 200                 205
   ggt att acc aga atg atc cta gaa gca aca aag ccg ccc att gta gta    672
   Gly Ile Thr Arg Met Ile Leu Glu Ala Thr Lys Pro Pro Ile Val Val
       210                 215                 220
   cca ata ttt gct aca ggg ttt gaa aaa ata gca tcc gaa gca gtc aca    720
   Pro Ile Phe Ala Thr Gly Phe Glu Lys Ile Ala Ser Glu Ala Val Thr
   225                 230                 235                 240
   gat tca atg ttt aga caa att cta cca aga aac ttt ggc tct gaa ata    768
   Asp Ser Met Phe Arg Gln Ile Leu Pro Arg Asn Phe Gly Ser Glu Ile
                   245                 250                 255
   aat gtt acc ata ggg gat cct tta aat gat gat tta atc gac agg tat    816
   Asn Val Thr Ile Gly Asp Pro Leu Asn Asp Asp Leu Ile Asp Arg Tyr
               260                 265                 270
   aga aaa gaa tgg aca cat ttg gtt gaa aaa tac tat gat ccc aaa aat    864
   Arg Lys Glu Trp Thr His Leu Val Glu Lys Tyr Tyr Asp Pro Lys Asn
           275                 280                 285
   cct aac gac ctc tct gac gaa ttg aaa tat ggt aaa gag gcg caa gat    912
   Pro Asn Asp Leu Ser Asp Glu Leu Lys Tyr Gly Lys Glu Ala Gln Asp
       290                 295                 300
   tta aga agc aga tta gcc gct gaa ctg aga gcc cat gtt gct gaa att    960
   Leu Arg Ser Arg Leu Ala Ala Glu Leu Arg Ala His Val Ala Glu Ile
   305                 310                 315                 320
   aga aat gaa gtt cgc aaa tta cca cgc gaa gac cct agg ttc aaa tcc    1008
   Arg Asn Glu Val Arg Lys Leu Pro Arg Glu Asp Pro Arg Phe Lys Ser
                   325                 330                 335
   ccc tca tgg tgg aag cgg ttc aac acc acg gaa ggt aaa tcg gac cca    1056
   Pro Ser Trp Trp Lys Arg Phe Asn Thr Thr Glu Gly Lys Ser Asp Pro
               340                 345                 350
   gat gtt aaa gtc att ggc gaa aat tgg gca ata agg agg atg caa aag    1104
   Asp Val Lys Val Ile Gly Glu Asn Trp Ala Ile Arg Arg Met Gln Lys
           355                 360                 365
   ttt ctg cct cca gag ggt aaa cca aag ggt aag gat gat tga              1146
   Phe Leu Pro Pro Glu Gly Lys Pro Lys Gly Lys Asp Asp
       370                 375                 380
<210> 2
   <211> 381
   <212> PRT
   <213> Saccharomyces cerevisiae
<400> 2
   Met Ser Phe Arg Asp Val Leu Glu Arg Gly Asp Glu Phe Leu Glu Ala
   1               5                   10                  15
   Tyr Pro Arg Arg Ser Pro Leu Trp Arg Phe Leu Ser Tyr Ser Thr Ser
               20                  25                  30
   Leu Leu Thr Phe Gly Val Ser Lys Leu Leu Leu Phe Thr Cys Tyr Asn
           35                  40                  45
   Val Lys Leu Asn Gly Phe Glu Lys Leu Glu Thr Ala Leu Glu Arg Ser
       50                  55                  60
   Lys Arg Glu Asn Arg Gly Leu Met Thr Val Met Asn His Met Ser Met
   65                  70                  75                  80
   Val Asp Asp Pro Leu Val Trp Ala Thr Leu Pro Tyr Lys Leu Phe Thr
                   85                  90                  95
   Ser Leu Asp Asn Ile Arg Trp Ser Leu Gly Ala His Asn Ile Cys Phe
               100                 105                 110
   Gln Asn Lys Phe Leu Ala Asn Phe Phe Ser Leu Gly Gln Val Leu Ser
           115                 120                 125
   Thr Glu Arg Phe Gly Val Gly Pro Phe Gln Gly Ser Ile Asp Ala Ser
       130                 135                 140
   Ile Arg Leu Leu Ser Pro Asp Asp Thr Leu Asp Leu Glu Trp Thr Pro
   145                 150                 155                 160
   His Ser Glu Val Ser Ser Ser Leu Lys Lys Ala Tyr Ser Pro Pro Ile
                   165                 170                 175
   Ile Arg Ser Lys Pro Ser Trp Val His Val Tyr Pro Glu Gly Phe Val
               180                 185                 190
   Leu Gln Leu Tyr Pro Pro Phe Glu Asn Ser Met Arg Tyr Phe Lys Trp
           195                 200                 205
   Gly Ile Thr Arg Met Ile Leu Glu Ala Thr Lys Pro Pro Ile Val Val
       210                 215                 220
   Pro Ile Phe Ala Thr Gly Phe Glu Lys Ile Ala Ser Glu Ala Val Thr
   225                 230                 235                 240
   Asp Ser Met Phe Arg Gln Ile Leu Pro Arg Asn Phe Gly Ser Glu Ile
                   245                 250                 255
   Asn Val Thr Ile Gly Asp Pro Leu Asn Asp Asp Leu Ile Asp Arg Tyr
               260                 265                 270
   Arg Lys Glu Trp Thr His Leu Val Glu Lys Tyr Tyr Asp Pro Lys Asn
           275                 280                 285
   Pro Asn Asp Leu Ser Asp Glu Leu Lys Tyr Gly Lys Glu Ala Gln Asp
       290                 295                 300
   Leu Arg Ser Arg Leu Ala Ala Glu Leu Arg Ala His Val Ala Glu Ile
   305                 310                 315                 320
   Arg Asn Glu Val Arg Lys Leu Pro Arg Glu Asp Pro Arg Phe Lys Ser
                   325                 330                 335
   Pro Ser Trp Trp Lys Arg Phe Asn Thr Thr Glu Gly Lys Ser Asp Pro
               340                 345                 350
   Asp Val Lys Val Ile Gly Glu Asn Trp Ala Ile Arg Arg Met Gln Lys
           355                 360                 365
   Phe Leu Pro Pro Glu Gly Lys Pro Lys Gly Lys Asp Asp
    370                 375                 380
<210> 3
   <211> 1374
   <212> DNA
   <213> Arabidopsis thaliana
<220>
   <221> CDS
   <222> (5)..(1348)
<400> 3
   caga atg gga att cat ttt gtt gac aag gca gat cta tgg aag agt gca    49
        Met Gly Ile His Phe Val Asp Lys Ala Asp Leu Trp Lys Ser Ala
        1               5                   10                  15
   ctg ttg ttc aat ctt aaa ctt cgt gat cga ttt cga atc gcc gtc gat     97
   Leu Leu Phe Asn Leu Lys Leu Arg Asp Arg Phe Arg Ile Ala Val Asp
                   20                  25                  30
   gat cac cgt ggt cga gct acg gtt ttc tca ccg gat ggt tgc ttc tct     145
   Asp His Arg Gly Arg Ala Thr Val Phe Ser Pro Asp Gly Cys Phe Ser
               35                  40                  45
   tcc act atc cac cgc tgg gtg act cga ttc cgg aac ttt cgc cgg gag     193
   Ser Thr Ile His Arg Trp Val Thr Arg Phe Arg Asn Phe Arg Arg Glu
           50                  55                  60
   tct ctc cct tct cca ccg gct ttt tat cgc aga cga gtt tct aag gac     241
   Ser Leu Pro Ser Pro Pro Ala Phe Tyr Arg Arg Arg Val Ser Lys Asp
       65                  70                  75
   tta acg gca gaa gaa gag tct gct ctt ttc cgg atg cta caa act gtg     289
   Leu Thr Ala Glu Glu Glu Ser Ala Leu Phe Arg Met Leu Gln Thr Val
   80                  85                  90                  95
   gct gtt ccc ctt att gga aat gct tgt cat gtt ttc atg aat ggt ttt     337
   Ala Val Pro Leu Ile Gly Asn Ala Cys His Val Phe Met Asn Gly Phe
                   100                 105                 110
   aac cgt gtt cag gta tat ggt tta gag aaa ttg cat gat gct tta ctc     385
   Asn Arg Val Gln Val Tyr Gly Leu Glu Lys Leu His Asp Ala Leu Leu
               115                 120                 125
   aac aga cca aag aac aag cct ctt gta acg gtg agc aat cat gtt gca     433
   Asn Arg Pro Lys Asn Lys Pro Leu Val Thr Val Ser Asn His Val Ala
           130                 135                 140
   tct gtg gat gat cca ttt gtc att gct tca tta ctt cca cct aaa ttt     481
   Ser Val Asp Asp Pro Phe Val Ile Ala Ser Leu Leu Pro Pro Lys Phe
       145                 150                 155
   cta ctt gat gcc cgt aat ttg agg tgg acg ctc tgt gct aca gat aga     529
   Leu Leu Asp Ala Arg Asn Leu Arg Trp Thr Leu Cys Ala Thr Asp Arg
   160                 165                 170                 175
   tgc ttt aaa aac cct gta act tca gct ttc tct cga tct gtc aaa gtt     577
   Cys Phe Lys Asn Pro Val Thr Ser Ala Phe Ser Arg Ser Val Lys Val
                   180                 185                 190
   ttg cca att tct cgt ggt gaa gga att tat cag cag gga atg gac att     625
   Leu Pro Ile Ser Arg Gly Glu Gly Ile Tyr Gln Gln Gly Met Asp Ile
               195                 200                 205
   gcg att tcg aaa ttg aac aac gga ggg tgg gtt cac atc ttt cca gaa     673
   Ala Ile Ser Lys Leu Asn Asn Gly Gly Trp Val His Ile Phe Pro Glu
           210                 215                 220
   ggc agt cgc tcc cgg gat ggt gga aag act atg ggc tca gcg aag agg     721
   Gly Ser Arg Ser Arg Asp Gly Gly Lys Thr Met Gly Ser Ala Lys Arg
       225                 230                 235
   ggt atc gga agg ttg att ttg gac gca gat act ctc cct atg gtt gtt     769
   Gly Ile Gly Arg Leu Ile Leu Asp Ala Asp Thr Leu Pro Met Val Val
   240                 245                 250                 255
   cct ttt gtg cat act ggt atg cag gat ata atg cca gtt gga gct agt     817
   Pro Phe Val His Thr Gly Met Gln Asp Ile Met Pro Val Gly Ala Ser
                   260                 265                 270
   gtt cca cgg att ggc aag aca gtg aca gtg atc att gga gac cct att     865
   Val Pro Arg Ile Gly Lys Thr Val Thr Val Ile Ile Gly Asp Pro Ile
               275                 280                 285
   cat ttt aat gac att ctc agc act gaa gga gcc caa cac gtc tca agg     913
   His Phe Asn Asp Ile Leu Ser Thr Glu Gly Ala Gln His Val Ser Arg
           290                 295                 300
   aaa cac ctg tat gac gcc gtt tcg tcc aga att gga caa aga ctg tac     961
   Lys His Leu Tyr Asp Ala Val Ser Ser Arg Ile Gly Gln Arg Leu Tyr
       305                 310                 315
   gat tta aaa gca caa gtt gat aga gta tat ata gaa caa caa tct atg    1009
   Asp Leu Lys Ala Gln Val Asp Arg Val Tyr Ile Glu Gln Gln Ser Met
   320                 325                 330                 335
   atg tca cac aat gcc aaa aca ccc tcg gac cgt gct gct gag atc ttt    1057
   Met Ser His Asn Ala Lys Thr Pro Ser Asp Arg Ala Ala Glu Ile Phe
                   340                 345                 350
   cat aga gtc gat tgg gac tca ttt ggg atg gga gca caa ttt tca gaa    1105
   His Arg Val Asp Trp Asp Ser Phe Gly Met Gly Ala Gln Phe Ser Glu
               355                 360                 365
   gaa tca tca ccc agt agt aaa ccg att ggc caa agc gat gat cgc att    1153
   Glu Ser Ser Pro Ser Ser Lys Pro Ile Gly Gln Ser Asp Asp Arg Ile
           370                 375                 380
   gtc aga tct ccc aaa agg aga gtc tca ccc gaa gga ggg gtc agc ttg    1201
   Val Arg Ser Pro Lys Arg Arg Val Ser Pro Glu Gly Gly Val Ser Leu
       385                 390                 395
   aag att aag aag ctc atg gac tca acc gag atg atg ggg ttt gcg gct    1249
   Lys Ile Lys Lys Leu Met Asp Ser Thr Glu Met Met Gly Phe Ala Ala
   400                 405                 410                 415
   aga ggg tta cta atg aac gag tac aag agt cgg gtt gaa tct gcc aat    1297
   Arg Gly Leu Leu Met Asn Glu Tyr Lys Ser Arg Val Glu Ser Ala Asn
                   420                 425                 430
   gtt ggt agg cct tta aag gct tgg aga gag tat ttt atg aac cgt gga    1345
   Val Gly Arg Pro Leu Lys Ala Trp Arg Glu Tyr Phe Met Asn Arg Gly
               435                 440                 445
   tta taacttgcaa gttaaactcc cgttag                                   1374
   Leu
<210> 4
   <211> 448
   <212> PRT
   <213> Arabidopsis thaliana
<400> 4
   Met Gly Ile His Phe Val Asp Lys Ala Asp Leu Trp Lys Ser Ala Leu
   1 5 10 15
   Leu Phe Asn Leu Lys Leu Arg Asp Arg Phe Arg Ile Ala Val Asp Asp
   20 25 30
   His Arg Gly Arg Ala Thr Val Phe Ser Pro Asp Gly Cys Phe Ser Ser
   35 40 45
   Thr Ile His Arg Trp Val Thr Arg Phe Arg Asn Phe Arg Arg Glu Ser
   50 55 60
   Leu Pro Ser Pro Pro Ala Phe Tyr Arg Arg Arg Val Ser Lys Asp Leu
   65 70 75 80
   Thr Ala Glu Glu Glu Ser Ala Leu Phe Arg Met Leu Gln Thr Val Ala
   85 90 95
   Val Pro Leu Ile Gly Asn Ala Cys His Val Phe Met Asn Gly Phe Asn
   100 105 110
   Arg Val Gln Val Tyr Gly Leu Glu Lys Leu His Asp Ala Leu Leu Asn
   115 120 125
   Arg Pro Lys Asn Lys Pro Leu Val Thr Val Ser Asn His Val Ala Ser
   130 135 140
   Val Asp Asp Pro Phe Val Ile Ala Ser Leu Leu Pro Pro Lys Phe Leu
   145 150 155 160
   Leu Asp Ala Arg Asn Leu Arg Trp Thr Leu Cys Ala Thr Asp Arg Cys
   165 170 175
   Phe Lys Asn Pro Val Thr Ser Ala Phe Ser Arg Ser Val Lys Val Leu
   180 185 190
   Pro Ile Ser Arg Gly Glu Gly Ile Tyr Gln Gln Gly Met Asp Ile Ala
   195 200 205
   Ile Ser Lys Leu Asn Asn Gly Gly Trp Val His Ile Phe Pro Glu Gly
   210 215 220
   Ser Arg Ser Arg Asp Gly Gly Lys Thr Met Gly Ser Ala Lys Arg Gly
   225                 230                 235                 240
   Ile Gly Arg Leu Ile Leu Asp Ala Asp Thr Leu Pro Met Val Val Pro
                   245                 250                 255
   Phe Val His Thr Gly Met Gln Asp Ile Met Pro Val Gly Ala Ser Val
               260                 265                 270
   Pro Arg Ile Gly Lys Thr Val Thr Val Ile Ile Gly Asp Pro Ile His
           275                 280                 285
   Phe Asn Asp Ile Leu Ser Thr Glu Gly Ala Gln His Val Ser Arg Lys
       290                 295                 300
   His Leu Tyr Asp Ala Val Ser Ser Arg Ile Gly Gln Arg Leu Tyr Asp
   305                 310                 315                 320
   Leu Lys Ala Gln Val Asp Arg Val Tyr Ile Glu Gln Gln Ser Met Met
                   325                 330                 335
   Ser His Asn Ala Lys Thr Pro Ser Asp Arg Ala Ala Glu Ile Phe His
               340                 345                 350
   Arg Val Asp Trp Asp Ser Phe Gly Met Gly Ala Gln Phe Ser Glu Glu
           355                 360                 365
   Ser Ser Pro Ser Ser Lys Pro Ile Gly Gln Ser Asp Asp Arg Ile Val
       370                 375                 380
   Arg Ser Pro Lys Arg Arg Val Ser Pro Glu Gly Gly Val Ser Leu Lys
   385                 390                 395                 400
   Ile Lys Lys Leu Met Asp Ser Thr Glu Met Met Gly Phe Ala Ala Arg
                   405                 410                 415
   Gly Leu Leu Met Asn Glu Tyr Lys Ser Arg Val Glu Ser Ala Asn Val
               420                 425                 430
   Gly Arg Pro Leu Lys Ala Trp Arg Glu Tyr Phe Met Asn Arg Gly Leu
           435                 440                 445
<210> 5
   <211> 961
   <212> DNA
   <213> Arabidopsis thaliana
<220>
   <221> CDS
   <222> (38)..(889)
<400> 5
   ctggtctcgt ttctaattgg agagaacttt gaagcta atg ggg aag ata atg gaa   55
                                            Met Gly Lys Ile Met Glu
                                             1               5
   tgg gca gca aga tct gat cat ttg gga gga att cca agg aat act gtg    103
   Trp Ala Ala Arg Ser Asp His Leu Gly Gly Ile Pro Arg Asn Thr Val
               10                  15                  20
   ata atg gct gtt agt gca ttt gca aaa gca gta gca aat ctt tgc aat    151
   Ile Met Ala Val Ser Ala Phe Ala Lys Ala Val Ala Asn Leu Cys Asn
           25                  30                  35
   aaa agc tca gtt cac aat gca gat act ctt atg aat ctt gtc cag tca    199
   Lys Ser Ser Val His Asn Ala Asp Thr Leu Met Asn Leu Val Gln Ser
       40                  45                  50
   aga cca cct ggt gtt cct ctc atc act gtt agt aat cac atg tcg act    247
   Arg Pro Pro Gly Val Pro Leu Ile Thr Val Ser Asn His Met Ser Thr
   55                  60                  65                  70
   ttg gat gat cca gta atg tgg ggg gca ttt aag ggt ctc ctt tcc tta    295
   Leu Asp Asp Pro Val Met Trp Gly Ala Phe Lys Gly Leu Leu Ser Leu
                   75                  80                  85
   gat cca gaa ttg gct cgg tgg gtt ctt gct gca gag gat ata tgt ttc    343
   Asp Pro Glu Leu Ala Arg Trp Val Leu Ala Ala Glu Asp Ile Cys Phe
               90                  95                  100
   agg aac cct ata ttc tcc tac att ttc cgc act gga aaa tgt ata cct    391
   Arg Asn Pro Ile Phe Ser Tyr Ile Phe Arg Thr Gly Lys Cys Ile Pro
           105                 110                 115
   ata act aga ggt ggt gga atc tac caa gaa aac atg aat gaa gct ctc    439
   Ile Thr Arg Gly Gly Gly Ile Tyr Gln Glu Asn Met Asn Glu Ala Leu
       120                 125                 130
   cag cga tta aaa gat gga tct tgg ctg cat acc ttc cca gag gga aag    487
   Gln Arg Leu Lys Asp Gly Ser Trp Leu His Thr Phe Pro Glu Gly Lys
   135                 140                 145                 150
   gtg ttt caa gat gat gtt cct ata aga cga ctt aaa tgg gga act gca    535
   Val Phe Gln Asp Asp Val Pro Ile Arg Arg Leu Lys Trp Gly Thr Ala
                   155                 160                 165
   agc ctc atc gcc cgt tcc cca gtt acc cca atc gtt ttg cca ata att    583
   Ser Leu Ile Ala Arg Ser Pro Val Thr Pro Ile Val Leu Pro Ile Ile
               170                 175                 180
   cac cgt ggt ttt gag gag atg atg ccg gag aac tac aat aat gga cga    631
   His Arg Gly Phe Glu Glu Met Met Pro Glu Asn Tyr Asn Asn Gly Arg
           185                 190                 195
   aga cca ctg gta ccg ttg ccg aac aaa cac ctt aaa gtt gtg gtt ggt    679
   Arg Pro Leu Val Pro Leu Pro Asn Lys His Leu Lys Val Val Val Gly
       200                 205                 210
   gaa cca att gag ttt gat gtt cca atg atg gtt gag act gct gtc ttg    727
   Glu Pro Ile Glu Phe Asp Val Pro Met Met Val Glu Thr Ala Val Leu
   215                 220                 225                 230
   gac tcc cgc cat gta acc cct cct ctt caa gaa gtg aaa tgg cct gtc    775
   Asp Ser Arg His Val Thr Pro Pro Leu Gln Glu Val Lys Trp Pro Val
                   235                 240                 245
   ctc act tct gct ggc caa gtg cta gac gaa act gct cag aga cac ctc    823
   Leu Thr Ser Ala Gly Gln Val Leu Asp Glu Thr Ala Gln Arg His Leu
               250                 255                 260
   tac ata gct ctg tcc gag aag att caa tcc tcc ttg gaa aca ttg aga    871
   Tyr Ile Ala Leu Ser Glu Lys Ile Gln Ser Ser Leu Glu Thr Leu Arg
           265                 270                 275
   ctc tta gcc aag cgg ttg tgacttccgc cgggaaactt catatcaaaa           919
   Leu Leu Ala Lys Arg Leu
       280
   tccggttcac aacttttaaa gttccggttt agattcgcca tg                     961
<210> 6
   <211> 284
   <212> PRT
   <213> Arabidopsis thaliana
<400> 6
   Met Gly Lys Ile Met Glu Trp Ala Ala Arg Ser Asp His Leu Gly Gly
    1               5                  10                  15
   Ile Pro Arg Asn Thr Val Ile Met Ala Val Ser Ala Phe Ala Lys Ala
               20                  25                  30
   Val Ala Asn Leu Cys Asn Lys Ser Ser Val His Asn Ala Asp Thr Leu
           35                  40                  45
   Met Asn Leu Val Gln Ser Arg Pro Pro Gly Val Pro Leu Ile Thr Val
       50                  55                  60
   Ser Asn His Met Ser Thr Leu Asp Asp Pro Val Met Trp Gly Ala Phe
   65                  70                  75                  80
   Lys Gly Leu Leu Ser Leu Asp Pro Glu Leu Ala Arg Trp Val Leu Ala
                   85                  90                  95
   Ala Glu Asp Ile Cys Phe Arg Asn Pro Ile Phe Ser Tyr Ile Phe Arg
               100                 105                 110
   Thr Gly Lys Cys Ile Pro Ile Thr Arg Gly Gly Gly Ile Tyr Gln Glu
           115                 120                 125
   Asn Met Asn Glu Ala Leu Gln Arg Leu Lys Asp Gly Ser Trp Leu His
       130                 135                 140
   Thr Phe Pro Glu Gly Lys Val Phe Gln Asp Asp Val Pro Ile Arg Arg
   145                 150                 155                 160
   Leu Lys Trp Gly Thr Ala Ser Leu Ile Ala Arg Ser Pro Val Thr Pro
                   165                 170                 175
   Ile Val Leu Pro Ile Ile His Arg Gly Phe Glu Glu Met Met Pro Glu
               180                 185                 190
   Asn Tyr Asn Asn Gly Arg Arg Pro Leu Val Pro Leu Pro Asn Lys His
           195                 200                 205
   Leu Lys Val Val Val Gly Glu Pro Ile Glu Phe Asp Val Pro Met Met
       210                 215                 220
   Val Glu Thr Ala Val Leu Asp Ser Arg His Val Thr Pro Pro Leu Gln
   225                 230                 235                 240
   Glu Val Lys Trp Pro Val Leu Thr Ser Ala Gly Gln Val Leu Asp Glu
                   245                 250                 255
   Thr Ala Gln Arg His Leu Tyr Ile Ala Leu Ser Glu Lys Ile Gln Ser
               260                 265                 270
   Ser Leu Glu Thr Leu Arg Leu Leu Ala Lys Arg Leu
           275                 280
<210> 7
   <211> 403
   <212> DNA
   <213> Zea mays
<220>
   <221> misc_feature
   <222> (1)..(403)
<400> 7
   ccgctcctca ccgtcagcaa tcacatgtcc acgttagatg acccgcttat gttggcgatt 60
   caagggtttt ccaacttcgg atgcaaagct tggaaggtgg gtgctgacag cagaagatat 120
   atgcttcaag aatgtagtca tgtcctacat gtttcgactt gggaaatgca taccaatcac 180
   gagagggggt gggatttatc aagaacacat gaatgaagcc cttgacgtgc ttagaaatgg 240
   aggctggctg catacattcc ctgaaggaaa aatagcccaa gaagatcagc cgattagaag 300
   attgaagtgg ggaactgcca gtcttattgt ccgagcacct ataactccaa tagttttgcc 360
   aattgttcac tctggtttcg aaaaggtcat gccagaaaac tcg                  403
<210> 8
   <211> 423
   <212> DNA
   <213> Glycine max
<220>
   <221> misc_feature
   <222> (1)..(423)
<400> 8
   ttcccgggtc gaccaccatt ggcgccgccg acaccaccac acctttatcc cctctgccga 60
   tggctacttc tcctccacca ttcaacgctg cctcagccga tttcgtgatt tccgaaggga 120
   ttcattgcct tcttccacct ctttctatcg taaacgagtg attaaggatt tcagttctga 180
   ggaagattca gctcttgttc gtacgatgca agctgttgcg gttcctgttc ttggaaatgt 240
   ctgtcacgtg tttatgaacg gattaaacca ggtgcaggtg tatggtttag aaaaactgca 300
   ctccgcgttg ctgcatagac ctaagggcaa acctcttctt acggtcagca atcatgttgc 360
   ttccatggat gatccgcttg ttatcgcttc gctgcttcct tcgagtgttc tcttggatgc 420
   tan                                                               423
<210> 9
   <211> 408'
   <212> DNA
   <213> Glycine max
<220>
   <221> misc_feature
   <222> (1)..(408)
<220>
   <221> misc_feature
   <222> (1)..(408)
<400> 9
   tttttatcaa ccaacgcaac atgtcacgga cgatggagtg ggcggcgagg gcggagcacc 60
   tccgcggcat tcccagaaaa ctcgtgattg cggcggtggg cggattcgcc aaaacggtgt 120
   cgtctttcct caacaccgcc gatgttcaca acggcgacac tctcctccgc ctcgtccgct 180
   ccagacccca ccgcgtcccc ctcattaccg ttagcaatca catgtccact ttggatgatc 240
   cggttatgtg ggggttcaag ggttttccta tcttcgacac caacttagct cgctgggttc 300
   tcgctgccga agatatttgc ttcaggaatg ccctctattc ctatattttt cggctgcata 360
   cttttccaga aggaaaagtg tatcaagaag atgcacctat aaggcaat 408
<210> 10
   <211> 368
   <212> DNA
   <213> Linum usitatissimum
<220>
   <221> misc_feature
   <222> (1)..(368)
<400> 10
   cggttagcaa ccatgttgct tctgtcgatg acccatttgt gattgcttca ttgctaccac 60
   caagagtact tttggatgct cagaacttga ggtggacact ttgcgcaagg atcgctgttt 120
   taggaatccc gtcacttctg cattcttaag actgtcaaag tcttgcccct ctctcgtggt 180
   catggagttt atcagaaggg tatggacacg gcaattgcga acgtgaacac tggtggctgg 240
   gttcacatct tcccggaggt agccgttcta aggatggtgg gaaaactatg gggtctatta 300
   aaagaggtgt tggaaggttg gtacttgatg ctgatacttc tccccattgt agtcccggtc 360
   gtgcacac 368
<210> 11
   <211> 376
   <212> DNA
   <213> Linum usitatissimum
<220>
   <221> misc_feature
   <222> (1)..(376)
<400> 11
   tcgtagttag agaacctcag atggctggga ttacaaggaa tgcagtgttt gtgaccgtcg 60
   gtgcctttgc taaggcagtg agtagtcttc tgaacaatac atcagtccac aatgcagaca 120
   ctctacttcg cctagttcga tctcggccgc ctggtgtacc tctcatcact gttagcaatc 180
   acatgtcaac gttagatgat cctctgatgt ggggattcaa gggattccca atcatgggat 240
   gcgaaattgt ttcgatgggt atgggctgct gaagacatct gtttcaggaa ttcttttcat 300
   tcttacttct ttcgcatggg gaaatgtatt cccattacaa gaggtggggg aatttatcgg 360
   agccacatga atgaag 376
<210> 12
   <211> 418
   <212> DNA
   <213> Linum usitatissimum
<220>
   <221> misc_feature
   <222> (1)..(418)
<400> 12
   ccgcctggtg tacctctcat cactgttagc aatcacatgt caacgttaga tgatcctctg 60
   atgtggggat tcaagggatt cccaatcatg aatgcgaaat tgtttcgatg ggtattagct 120
   gctgaagaca tctgtttcag gaattctttt cattcttact tctttcgcat ggggaaatgt 180
   attcccatta caagaggtgg tggaatttat caaagccaca tgaatgaagc tcttcagcgc 240
   ttgagcaatg gtgattggct gcacacattc cctgagggaa aggtcaacca agaaattgga 300
   cctataagac gattgaaatg gggaactgcc agtctcatcg tccgtgcccc tgttacaccg 360
   atagtattac ccattgttca tcgtggcttt caagaggtga tgccagagaa ctacctat 418
<210> 13
   <211> 445
   <212> DNA
   <213> Glycine max
<220>
   <221> misc_feature
   <222> (1)..(445)
<400> 13
   ttccgggtcg accgccattg gcgccgccgc cactaccacc acacctttat cccctccgcc 60
   gatggctact tctcctccac cattcaacgc tggctcagcc gatttcgtga tttccgcaga 120
   gactcgttgc cgtcgtccac ctctttttat cgcaaacgag tgattaagga tttcagttct 180
   gaagaagatt caactcttgt tcgtatgatg caagctgttg cggttcctgt tcttggaaat 240
   gtctgtcacg tgtttatgaa cggattaaac agtgtgcagg tatatggttt agaaaaactg 300
   cactccgctt tactgcaaag acctaaagga aaacctcttc ttacggtcag caatcatgtt 360
   gcttccatgg atgatcctct tgttattgct tcgctgcttc ctccgagtgt tcttttggac 420
   gctaggaatc tcagatggac gcttc 445
<210> 14
   <211> 361
   <212> DNA
   <213> Hordeum vulgare
<220>
   <221> misc_feature
   <222> (1)..(361)
<400> 14
   ggaatcggtg atcctccgca tggttcaagc tgtggcggtt cctctattgg gaaacatgtg 60
   ctacgtgttc atgaatggcc tcaatcgcgt tcaggttcat ggcctggaga agctgcacaa 120
   ggcattgctt gagaggccta aggacaagcc cctagtaacg gttagcaacc atgttgcttc 180
   tgtcgatgac ccatttgtga ttgcttcatt gctaccacca agagtacttt tggatgctca 240
   gaacttgagg tggacacttt gcgcaacgat cgctgtttta ggaatcccgt cacttctgca 300
   ttctttaaga ctgtcaaagt cttgcccctc tctcgtggtc atggagttta tcagaagggt 360
   a 361
<210> 15
   <211> 472
   <212> DNA
   <213> Brassica napus
<220>
   <221> misc_feature
   <222> (1)..(472)
<400> 15
   tgtatcggaa tttccgggtc gacgaccacc gccggagagc cgcggtttta tatacggacg 60
   gttacttctc ttcctccatc caccgcttgg ctgctcgatt gcggaacttc cgccgcgagt 120
   ctctcccttc tgcccccgct ttttatcgca gaagagtacc taaagatttg acggcagaag 180
   aagagtctgc tatcttccgg atgcttcaag ctgtggctgt tccacttatt ggaaacgctt 240
   gtcatgtttt catgaatggt cttaaccgtg ttcaggtgta tggtttagag aagttgcatg 300
   atgctctgct caacaggcca aagaacaagc ctctcgtaac ggttagcaat catgtggcat 360
   ccttggatga tccatttgtc attgcttcgt tacttccgcc taagcttcta ctcgatgctc 420
   gtaatttgag gtggacgctt tgtgctacag atagatgctt taagaaccct gt         472
<210> 16
   <211> 412
   <212> DNA
   <213> Brassica napus
<220>
   <221> misc_feature
   <222> (1)..(412)
<400> 16
   tttagatgat ccagtaatgt ggggagggtt caaggcgtct tctttcctta gatccagagc 60
   tggctcgatg ggttcttgct gcagaggaca tttgtttcaa gaaccctgtc ttctcctaca 120
   tcttccgcac tggcaagtgt atacctataa ctagaggtgg tggaatctac caagaacaca 180
   tgagtgaagc tctcgagcga ttaaaagatg gatcttggtt gcataccttc ccagagggca 240
   aggtgtttca agaagatgtg cctataagac gacttaaatg gggaaccgca agcctcatcg 300
   cccgttgccc agtcacccca atcgtcttgc caataattca ccgtggtttg acgagatgaa 360
   tgccgagagt acatttatgg aaaangacca ccgtacccgt tgggaacaaa an         412
<210> 17
   <211> 410
   <212> DNA
   <213> Brassica napus
<220>
   <221> misc_feature
   <222> (1)..(410)
<400> 17
   tttagatgat ccagtaatgt ggggagggtt caagggtctt ctttccttag atccagagct 60
   ggctcgatgg gttcttgctg cagaggacat ttgtttcaag aaccctgtct tctcctacat 120
   cttccgcact ggcaagtgta tacctataac tagaggtggt ggaatctacc aagaacacat 180
   gagtgaagct ctcgagcgat taaaagatgg atcttggttg catacctccc cagagggcaa 240
   ggtgtttcaa gaagatgtgc ctataagacg acttaaatgg ggaaccgcaa gcctcatcgc 300
   ccgttgccca gtcacccaaa tcgtcttgcc aatatttcac cgtggttttg acaacatgat 360
   gcccgaaaat gtccttttat ggaagaatga caaccgtacc tgtggggaan            410
<210> 18
   <211> 420
   <212> DNA
   <213> Glycine max
<220>
   <221> misc_feature
   <222> (1)..(420)
<400> 18
   gccattggcg ccgccgacac aaccaccaca cctttatccc ctccgccgat ggctacttct 60
   cctccaccat tcaacgctgg ctcagccgat ttcgtgattt ccgcagagac tcgttgccgt 120
   cgtccacctc tttttatcgc atacgagtga ttaaggattt cagttctgaa gaagattcaa 180
   ctcttgttcg tatgatgcaa gctgttgcgg ttcctgttct tggaaatgtc tgtcacgtgt 240
   ttatgaacgg attaaacagt gtgcaggtat atggtttaaa aaaactgcac tccgctttac 300
   tgcaaagacc taaaggaaaa cctcttctta cggtcagcaa tcatgttgct tccatggatg 360
   atcctcttgt tattgcttcg ctgcttcctc cgagtgttct tttggacgct aggaatctcn 420
<210> 19
   <211> 490
   <212> DNA
   <213> Brassica napus
<220>
   <221> misc_feature
   <222> (1)..(490)
<400> 19
   aattcctggg tcgacgattt cgtcccgaga tggtggcaag actatgggct cagcaaaaag 60
   aggtattgga aggttgattt tggacgcaga taccctccct atggttgttc catttgtgca 120
   tactggtatg caagatataa tgcctatagg agccagtgtt ccacggattg gcaaaacagt 180
   gacagtgatc attggagatc ctattccctt taatgacctt gtagacactg aaggagccaa 240
   acacgtttca aggaagcagt tgtatgacgc tgtatcttcc aggataggac aaagattaca 300
   ccagttaaag caacaggttg ataaagtatc tctgggagca caatattcag aagaatcacc 360
   agcccttctt ggtaaacaaa tttcccaaac cgatgtncgt ctcaatggtt tggactggca 420
   tgttcctaaa agggattgcc atccgaagga agcatcagcc tgaaggttaa gaggtttatg 480
   gactctacag                                                        490
<210> 20
   <211> 386
   <212> DNA
   <213> Zea mays
<220>
   <221> misc_feature
   <222> (1)..(386)
<400> 20
   cgtgcttaga aatggaggct ggctgcatac attccctgaa ggaaaaatag cccaagaaga 60
   tcagccgatt agaagattga agtggggaac ggccagtctt attgtccgag cacctataac 120
   tccaatagtt ttgccaattg ttcactctgg tttcgaaaag gtcatgccag aaaactcgtt 180
   ctttggacgg cgaccaccgg tgccactctg cagtaagaag atagacatca ttgttggaga 240
   gccaatagag tttgacttgc caagcttgaa gcaagaagca tcaacggtac cccatgactc 300
   atcctctgaa cggaaggggt ggccggccat tacaccagat gggctggacg aggccgccca 360
   gagatggctt taccagaaga tgtcag 386
<210> 21
   <211> 429
   <212> DNA
   <213> Brassica napus
<220>
   <221> misc_feature
   <222> (1)..(429)
<400> 21
   ctcgggtcga cgattccgta cggtcttaac cgagttcagg tgtatggttt atagaagctg 60
   tatgatgctc tgctcaacag gccaaagaac aagcctctcg taacggctaa caatgatgtg 120
   gcatccttgg atgatccatt cgccattgct tcattactat ccgcctaagc ttctactctg 180
   atgctcgtaa tttgaggtgg acgctttgtg ctacagatag atgctttaag aaccctgtaa 240
   cttcagcttt ctttcgatca ttcaaagttt tgccagcttc tcgcggtgaa ggaatctatc 300
   agcagggaat ggacatcgcg acgtcgaaat tgaataatgg aggatgggtt cacatatttc 360
   cagaaggcag acggtaccga gatggtggct agactatggg ttcacgcaat agaggtattg 420
   gaatgttgt 429
<210> 22
   <211> 436
   <212> DNA
   <213> Brassica napus
<220>
   <221> misc_feature
   <222> (1)..(436)
<400> 22
   tctagatgac ccactattgt ggggagggct ccagggtctt atttccttag atccaaagct 60
   ggctcgatgg gatcttgctg cagaggacat ttgtttcaat aaccctgtct tctcctacat 120
   tttccgcact gacacgcgta tacctataac tagaggtggt ggaatctacc aagaacacat 180
   gagtgaagct ctagagcgat taatagatgg atcttgcacg gcaaggcgtt tcaagaagat 240
   gtgcctataa gacgacttaa atggggaacc gcaagcctca tcagccgttg cccagtcacc 300
   ccaatcgtct tgccaataat tcaccgtggt tctgacgaga tgatgccgga gaagtacatt 360
   tatggaagaa taccaccgtt accgctgtgg aacaaaaacc ttaaagtagt tgttggtgaa 420
   ccaatcagag ttgatg 436
<210> 23
   <211> 423
   <212> DNA
   <213> Brassica napus
<220>
   <221> misc_feature
   <222> (1)..(423)
<400> 23
   ggatgatcca tttgtcattg cttcgttact tccgcctaag cttctactcg atgctcgtaa 60
   tttgaggtgg acgctttgtg ctacagatag atgcttcaaa aaccctgtaa cttcagcttt 120
   ctttcgatcc gtcaaggttt tgccagtttc tcgcggtgaa ggaatttatc agcagggaat 180
   ggacattgcg atttcgaaat tgaataatgg aggatgggtt cacatatttc cagaaggtag 240
   tcgctcccga gatggtggca agactatggg ctcagcaaaa agaggtattg gaaggttgat 300
   tttggacgca gataccctcc ctaatgttgt tccatttgtg catactggta tgcaagatat 360
   aatgcctata ggagccagtg ttccacggat tggcaaaaca gtgacagtga tcattggaga 420
   tcn                                                               423
<210> 24
   <211> 400
   <212> DNA
   <213> Oryza sativa
<220>
   <221> misc_feature
   <222> (1)..(400)
<400> 24
   gcgatggcat ctaccaaaag ggaatggaca tggcactttc aaagttgaac aatggtggat 60
   gggttcatat tttcccagaa ggaagtcgtt caaaggatgg agggaaaacc gtcgctcctg 120
   ccaagagagg tgttggaaga ttggtaatgg acgctgacag ccttccagtt gtaataccct 180
   ttgtccatac aggaatgcag gatataatgc ctgtcggaaa acgtattcca agagcaggca 240
   aaagggtgat tgtggttgtt ggtgatccaa tcaacttcaa cgaccttatc attgacaaca 300
   gcgatgaaac ccaacacatc tctagaggca ttttgtatga caaagcaaca gaaaggattg 360
   ggcagagact gcaggaactg aaagccgaag tcgatagatt                       400
<210> 25
   <211> 414
   <212> DNA
   <213> Brassica napus
<220>
   <221> misc_feature
   <222> (1)..(414)
<400> 25
   ggcagcaaga tctgatcact tgggaggaat cccaagaaaa actgtgataa cagccgttgg 60
   tgctttcgcg agagcagtag ctaatctttg caacaaaacc aaagttcaca atgcagatac 120
   tcttatgact cttgtccgtt cacgaccacc tggtgtccct ctcatcactt ttagatgatc 180
   cagtaatgtg gggagggttc aagggtcttc tttctttaga tccagagttg gctcgatggg 240
   tgcttgctgc tgaggatata tgtttcaaga actctttctt ctcctacatc ttccgcactg 300
   gcaagtgtat acctataact agaggtggtg gaatctatca agaacacatg agtgaagctc 360
   ttgaacgatt aaaagatgga tcttggttgc ataccttccc agaggggcag gtgg       414
<210> 26
   <211> 397
   <212> DNA
   <213> Brassica napus
<220>
   <221> misc_feature
   <222> (1)..(397)
<400> 26
   ctgcccccgc tttttatcgc agaagagtac ctaaagattt gacggcagaa gaagagtctg 60
   ctatcttccg gatgcttcaa gctgtggctg ttccacttat tggaaacgct tgtcatgttt 120
   tcatgaatgg tcttaaccgt gttcaggtgt atggtttgga gaagttgcat gatgctttac 180
   tcaacagacc aaagaacaag cctcttgtaa cggttagcaa tcatgtggcg tccttggatg 240
   atccatttgt cattgcttcg ttacttcctc ctaagcttct acttgatgct cgtaatctga 300
   ggtggacgct ttgtgctaca gatagatgct ttaagaaccc tgtaacttca gctttctttc 360
   gatccgtcaa agttttgcca gtttctcgcg gtgaagg                         397
<210> 27
   <211> 429
   <212> DNA
   <213> Brassica napus
<220>
   <221> misc_feature
   <222> (1)..(429)
<400> 27
   gaattcaacg tcgacgattt cgtcgatccg tcaaggtttt gccagtttct cgcggtgaag 60
   gaatttatca gcagggaatg gacattgcga tttcgaaatt gaataatgga ggatgggttc 120
   acatatttcc agaaggtagt cgctcccgag atggtggcaa gactatgggc tcagcaaaaa 180
   gaggtattgg aaggttgatt ttggacgcag ataccctccc tatggttgtt ccatttgtgc 240
   atactggtat gcaacatata atgcctatag gagccactgt tccacggatt gacaaaacag 300
   tgacagtgat cattggagat cctattccct ttagtgacct tgtagacact gaacgatcca 360
   aacacgtttc aaggaaccag gtttatgacc ctctatcgtt caggatcgac agcgattacc 420
   ctcctgcat                                                         429
<210> 28
   <211> 404
   <212> DNA
   <213> Brassica napus
<220>
   <221> misc_feature
   <222> (1)..(404)
<400> 28
   gttacttccg cctaagcttc tactcgatgc tcgtaatttg aggtggacgc tttgtgctac 60
   agatagatgc ttcaaaaacc ctgtaacttc agctttcttt cgatccgtca aggttttgcc 120
   agtttctcgc ggtgaaggaa tttatcagca gggaatggac attgcgattt cgaaattgaa 180
   taatggagga tgggttcaca tatttccaga aggtagtcgc tcccgatatg gtggcaagac 240
   tatgggctca gcaaaaagag gtattggaag gtgagtcata tatgccttta ctttcagcta 300
   ctttatgtaa tgcgtgtgta tggaccttat tataacacaa acaagcttgt gattcacttc 360
   tttgtgcaag atgatttctc tctcagatac catgcgtatg aatg                  404
<210> 29
   <211> 467
   <212> DNA
   <213> Brassica napus
<220>
   <221> misc_feature
   <222> (1)..(467)
<400> 29
   gaattctcgg gtcgacgata gtgcaaattt agatgatcca gtaatgtggg gagggttcaa 60
   ggtcttcttt ccttagatcc agagctggct cgatgggtac ttgctgcaga ggacatttgt 120
   ttcaagaacc ctgtcttctc ctacatcttc cgcactggca agtgtatacc tataactaga 180
   ggtggtggaa tctaccaaga acacatgagt gaagctctcg agcgattaaa agatggatct 240
   tggttgcata ccttcccaca gggcacggtg ttacacgatg atgtgcctag ctgacgactt 300
   acatggggaa ccggcggcct aatcccgcgt tgaccaacca cgccaattct cttgccaata 360
   tttcacggcg actgtgacga catcatgacg cagaaggcca tggatctata aacaccaccg 420
   ctacctctct tgatcaaaac cgtaaacgta gaggaggcta accctcn               467
<210> 30
   <211> 459
   <212> DNA
   <213> Brassica napus
<220>
   <221> misc_difference
   <222> (1)..(459)
<400> 30
   gatcaccgtg gtagagccgc ggttttatat acggcacggt atagcgttct cttctccatc 60
   caccgcttgg ctgctcgatt ccggaacttc cgccgcgagt ctctcccttc tgcccccgct 120
   ttttatcgca gaagagtacc taaagatttg acggcagaag aagagtctgc tatcttccgg 180
   atgcttcaag ctgtggctgt tccacttatt ggaaacgctt gtcatgtttt catgaatggt 240
   cttaaccgtg ttcaggtgta tggtttagag aagttgcatg atgctctgct caacaggcca 300
   aagaacaagc ctctcgtaac ggttagcaat catgtggcat ccttggatga tccatttgtc 360
   attgcttcgt tacttccgcc taagcttcta ctcgatgctc ggaatttgag gtggacgctt 420
   tgggctacac acagatggtt taccaaccct gtgcttccg                        459
<210> 31
   <211> 389
   <212> DNA
   <213> Glycine max
<220>
   <221> misc_feature
   <222> (1). (389)
<400> 31
   ggggtactgc gcccgcaatt cccggnccgg accaccattg gcgccgcgac accaccacac 60
   ctttatcccc tctgccgatg gctacttctc ctcgaccatt caacgctgcc tcagccgatt 120
   tcgtgatttc cgaaggtatt cattgccttc ttccacctct ttctatcgta aacgagtgat 180
   taaggatttc agttctgagg aagattcagc tcttgttcgg acgatgcaag ctgttgcggt 240
   tcctgttctt ggaaatgtct gtcacgtgtt tatgaacgga ttaaaccagg tgcaggtgta 300
   tggtttagaa aaactgcact ccgcgttgct gcatagacct aagggcaaac ctcttcttac 360
   ggtcagcaat catgttgctt ccatggatg 389
<210> 32
   <211> 400
   <212> DNA
   <213> Oryza sativa
<220>
   <221> misc_feature
   <222> (1)..(400)
<400> 32
   agaaaaactg cactccgctt tactgcaaag acctaaagga aaacctcttc ttacggtcag 60
   caatcatgtt gcttccatgg atgatcctct tgttattgct tcgctgcttc ctccgagtgt 120
   tcttttggac gctaggaatc tcagatggac gctttgcgca actgataggt gttttaaaaa 180
   ccctgtgact tctgcattct ttcgatcagt caaagttttg ccagtttctc gaggtgatgg 240
   catttatcaa gaaggaatgg acttggccat atcaaaattg aaccatggtg gttgggtcca 300
   gatattccca cacggcggtt gatccctcta tttttcaaaa tcagaaagtt aaaataaggg 360
   agggggcgtc gaaaaatcca agcggggagc gggccccttg 400
<210> 33
   <211> 449
   <212> DNA
   <213> Brassica napus
<220>
   <221> misc_feature
   <222> (1)..(449)
<400> 33
   aattcccggg tcgacgatca ccgtggcaga gccgcggttt tatatacgga cggttacttc 60
   tcctcctcca tccaccgctt ggctgctcga ttccggaact tccgccgcga gtctctccct 120
   tctgcccccg ctttttatcg cagaagagta cctaaagatt tgacggcaga agaagagtct 180
   gctatcttcc ggatgcttca agctgtggct gttccactta ttggaaacgc ttgtcatgtt 240
   ttcatgaatg gtcttaaccg tgttcaggtg tatggtttgg agaagttgca tgatgcttta 300
   ctcaacagac caaagaacaa gcctcttgta acggttagca atcatgtggc gtccttggat 360
   gatccatttg tcattgcttc gttacttcct cctaagcttc tacttgatgc tcgtaatctg 420
   aggtggacgc tntgtgctac agatagatg 449
<210> 34
   <211> 429
   <212> DNA
   <213> Oryza sativa
<220>
   <221> misc_feature
   <222> (1)..(429)
<400> 34
   ccgggatggt ggaaaaacca tgggctcttc caagagaggt gttgggaggt tagtcctgga 60
   tggagatagc atgcctgttg ttgtcccatt tgtacataca gggatgcagg agattatgcc 120
   tgtaggtgct aactttccca gaataggcaa gatggttaca gtgctcatag gtgatccgat 180
   caattttgat gatataattg aatttgacaa agacanaggc tcaaatgtgc ccagaagacg 240
   actatatgat gcagtagcat ctaaaattgg tgatcggttg cttgagatga aggtccaggt 300
   tgacactatc gcaattgtca agaaatgcag gtaccagaaa agtcctcaca cagactgacc 360
   gaccattaaa aaactgagcc aggtgattgg gactaatttg aatggacatc ttctggccgc 420
   agaaatgcc                                                         429
<210> 35
   <211> 449
   <212> DNA
   <213> Brassica napus
<220>
   <221> misc_feature
   <222> (1)..(449)
<400> 35
   aattcccggg tcgacgatca ccgtggcaga gccgcggttt tatatacgga cggttacttc 60
   tcctcctcca tccaccgctt ggctgctcga ttccggaact tccgccgcga gtctctccct 120
   tctgcccccg ctttttatcg cagaagagta cctaaagatt tgacggcaga agaagagtct 180
   gctatcttcc ggatgcttca agctgtggct gttccactta ttggaaacgc ttgtcatgtt 240
   ttcatgaatg gtcttaaccg tgttcaggtg tatggtttgg agaagttgca tgatgcttta 300
   ctcaacagac caaagaacaa gcctcttgta acggttagca atcatgtggc gtccttggat 360
   gatccatttg tcattgcttc gttacttcct cctaagcttc tacttgatgc tcgtaatctg 420
   aggtggacgc tntgtgctac agatagatg                                   449